# EUROPEAN PATENT APPLICATION

(11) **EP 4 768 910 A1**
(43) Date of publication of application: **01.07.2026**
(21) Application number: 24856371.0
(22) Date of filing: 13.08.2024
(51) Int. Cl.: G01N 33/48, G16H 10/40

(54) **IMAGE DISPLAY DEVICE, METHOD FOR OPERATING IMAGE DISPLAY DEVICE, AND OPERATION PROGRAM OF IMAGE DISPLAY DEVICE**

(30) Priority: 23.08.2023 JP 2023135821
(71) Applicant: FUJIFILM Corporation, Tokyo 106-8620 (JP)
(72) Inventor: TOMINAGA, Shunsuke, Ashigarakami-gun, Kanagawa 258-8577 (JP); INOMATA, Akira, Ashigarakami-gun, Kanagawa 258-8577 (JP)
(74) Representative: HGF
(86) International application number: PCT/JP2024/028917
(87) International publication number: WO 2025/041680

(57) **Abstract**

An image display device comprising a processor, in which the processor is configured to acquire a plurality of specimen images in which tissue specimens of a subject provided for an evaluation test of a candidate substance of a drug are imaged, receive designation of a criterion for setting a display priority of the plurality of specimen images from a user, set the display priority in accordance with the designated criterion, and perform control of displaying the plurality of specimen images on a display unit in accordance with the set display priority.

## Description

### BACKGROUND OF THE INVENTION

### 1. Field of the Invention

The technology of the present disclosure relates to an image display device, an operation method of an image display device, and an operation program of an image display device.

### 2. Description of the Related Art

In a process of pharmaceutical development, a test is performed in which a candidate substance of a drug (pharmaceutical) is administered to a subject such as a rat, and drug efficacy and toxicity of the candidate substance are evaluated. In such an evaluation test, a specimen image in which a tissue specimen (a brain specimen, a liver specimen, a heart specimen, or the like) of an organ collected by performing an autopsy on the subject is imaged is used. The specimen image is digitized to be a whole slide image (WSI). The specimen image is displayed on a display of a computer and is provided for browsing by a user such as a pathologist who is responsible for evaluating the candidate substance.

JP2022-525256A discloses a technique of setting a display priority of a specimen image using a machine learning model in order to exclude a tissue specimen having low quality or to quickly determine a need to produce an additional tissue specimen.

### SUMMARY OF THE INVENTION

Meanwhile, the subject is divided into a dose group in which the candidate substance is administered and a control group in which the candidate substance is not administered. Further, the dose group is divided into a high-dose group, a medium-dose group, a low-dose group, and the like according to the dose of the candidate substance. The organs serving as sources of the tissue specimens vary widely and include a brain, an esophagus, a stomach, a large intestine, a small intestine, a liver, a kidney, a spleen, a pancreas, a heart, a testis, an ovary, and the like. Therefore, the number of specimen images handled at once in the evaluation test is enormous.

In the related art, there has been a demand to organize and analyze such a large number of specimen images from the viewpoint desired by the user. In a case where the demand can be met, the improvement of the efficiency of evaluation and the improvement of the accuracy of evaluation are expected. However, in the technique disclosed in JP2022-525256A, the user cannot change a criterion for setting the display priority of the specimen image. Therefore, the set display priority does not always conform to the viewpoint desired by the user.

One embodiment according to the technology of the present disclosure provides an image display device, an operation method of an image display device, and an operation program of an image display device, which can set a display priority of a specimen image in accordance with a viewpoint desired by a user.

An image display device according to the present disclosure comprises a processor, in which the processor is configured to acquire a plurality of specimen images in which tissue specimens of a subject provided for an evaluation test of a candidate substance of a drug are imaged, receive designation of a criterion for setting a display priority of the plurality of specimen images from a user, set the display priority in accordance with the designated criterion, and perform control of displaying the plurality of specimen images on a display unit in accordance with the set display priority.

It is preferable that organs serving as sources of the tissue specimens are of a plurality of types, the plurality of types of organs are classified into a plurality of groups in advance, and the processor is configured to receive designation of the group as the designation of the criterion, specify types of the organs of the tissue specimens imaged in the specimen images, and set a display priority of the specimen images in which the tissue specimens of the organs belonging to the designated group are imaged to be higher than a display priority of the specimen images in which the tissue specimens of the organs belonging to a group other than the designated group are imaged.

It is preferable that the processor is configured to perform control of displaying the specimen images collectively for each group.

It is preferable that a plurality of the tissue specimens are imaged in one specimen image, and the processor is configured to identify the plurality of tissue specimens imaged in the one specimen image, and specify types of the organs of the identified tissue specimens.

It is preferable that the processor is configured to generate region images of the identified tissue specimens from the specimen image, and rearrange the region images in accordance with the set display priority.

It is preferable that the group is a group based on an organ system.

It is preferable that the group is a group based on knowledge of the user.

It is preferable that the processor is configured to receive designation of any one of a dose, a dosing period, or a dosing frequency of the candidate substance to the subject as the designation of the criterion, and set a display priority of the specimen images in which the tissue specimens of the subject having a relatively large dose, a relatively long dosing period, or a relatively high dosing frequency are imaged to be higher than a display priority of the specimen images in which the tissue specimens of the subject having a relatively small dose, a relatively short dosing period, or a relatively low dosing frequency are imaged.

It is preferable that the processor is configured to receive designation of a clinical test value of the subject as the designation of the criterion, and set a display priority of the specimen images in which the tissue specimens of the subject having a relatively large deviation of the clinical test value from a normal value are imaged to be higher than a display priority of the specimen images in which the tissue specimens of the subject having a relatively small deviation of the clinical test value from the normal value are imaged.

It is preferable that the processor is configured to derive a score representing a possibility that a morphological abnormality has occurred in the tissue specimens imaged in the specimen images, receive designation of the score as the designation of the criterion, and set a display priority of the specimen images in which the tissue specimens having a relatively high score and a relatively high possibility that the morphological abnormality has occurred are imaged to be higher than a display priority of the specimen images in which the tissue specimens having a relatively low score and a relatively low possibility that the morphological abnormality has occurred are imaged.

It is preferable that the processor is configured to extract a region in which the morphological abnormality is estimated to have occurred by using a machine learning model.

It is preferable that the score is a numerical value based on an area of the region in which the morphological abnormality is estimated to have occurred.

An operation method of an image display device according to the present disclosure comprises acquiring a plurality of specimen images in which tissue specimens of a subject provided for an evaluation test of a candidate substance of a drug are imaged, receiving designation of a criterion for setting a display priority of the plurality of specimen images from a user, setting the display priority in accordance with the designated criterion, and performing control of displaying the plurality of specimen images on a display unit in accordance with the set display priority.

An operation program of an image display device according to the present disclosure causes a computer to execute a process comprising acquiring a plurality of specimen images in which tissue specimens of a subject provided for an evaluation test of a candidate substance of a drug are imaged, receiving designation of a criterion for setting a display priority of the plurality of specimen images from a user, setting the display priority in accordance with the designated criterion, and performing control of displaying the plurality of specimen images on a display unit in accordance with the set display priority.

According to the technology of the present disclosure, it is possible to provide an image display device, an operation method of an image display device, and an operation program of an image display device, which can set a display priority of a specimen image in accordance with a viewpoint desired by a user.

### BRIEF DESCRIPTION OF THE DRAWINGS

FIG. 1 is a diagram showing a generation procedure of a specimen image, the specimen image, and an evaluation support apparatus.
FIG. 2 is a diagram showing a dose group and a control group.
FIG. 3 is a block diagram showing a computer constituting the evaluation support apparatus.
FIG. 4 is a block diagram showing a processing unit of a CPU of the evaluation support apparatus.
FIG. 5 is a diagram showing group information.
FIG. 6 is a diagram showing processing of an identification unit.
FIG. 7 is a diagram showing processing of a specifying unit.
FIG. 8 is a diagram showing processing of the specifying unit.
FIG. 9 is a diagram showing an image list display screen in an initial state.
FIG. 10 is a diagram showing a display designation screen.
FIG. 11 is a diagram showing processing of a setting unit.
FIG. 12 is a diagram showing processing of the setting unit.
FIG. 13 is a diagram showing an image list display screen after display designation.
FIG. 14 is a diagram showing a display example of a region image.
FIG. 15 is a flowchart showing a processing procedure of the evaluation support apparatus.
FIG. 16 is a flowchart showing a processing procedure of the evaluation support apparatus.
FIG. 17 is a flowchart showing a processing procedure of the evaluation support apparatus.
FIG. 18 is a diagram showing another example of the group information.
FIG. 19 is a diagram showing a display designation screen of a second embodiment.
FIG. 20 is a diagram showing processing of a setting unit of the second embodiment.
FIG. 21 is a diagram showing a display example of a region image of the second embodiment.
FIG. 22 is a diagram showing another example of the processing of the setting unit of the second embodiment.
FIG. 23 is a diagram showing still another example of the processing of the setting unit of the second embodiment.
FIG. 24 is a diagram showing a display designation screen of a third embodiment.
FIG. 25 is a diagram showing processing of a setting unit of the third embodiment.
FIG. 26 is a diagram showing a display example of a region image of the third embodiment.
FIG. 27 is a diagram showing a display designation screen of a fourth embodiment.
FIG. 28 is a block diagram showing a processing unit of a CPU of an evaluation support apparatus of the fourth embodiment.
FIG. 29 is a diagram showing an extraction model group.
FIG. 30 is a diagram showing a patch image obtained by subdividing the region image.
FIG. 31 is a diagram showing a state in which a feature amount is extracted from the patch image by an extraction model.
FIG. 32 is a diagram showing a configuration of the extraction model.
FIG. 33 is a diagram showing a process in a learning phase of an autoencoder.
FIG. 34 is a diagram showing a configuration of a past control group and a reference patch image for learning.
FIG. 35 is a diagram showing a state in which a reference feature amount is extracted from a reference patch image by the extraction model.
FIG. 36 is a diagram showing a graph in which the reference feature amount is plotted in a feature amount space and extraction reference information.
FIG. 37 is a diagram showing a distance between a position of the feature amount and a representative position of the reference feature amount.
FIG. 38 is a diagram showing processing of a derivation unit and a determination result.
FIG. 39 is a diagram showing processing of the derivation unit and a determination result.
FIG. 40 is a diagram showing processing of the derivation unit and a score.
FIG. 41 is a diagram showing processing of a setting unit of the fourth embodiment.
FIG. 42 is a diagram showing another example of the processing of the setting unit of the fourth embodiment.

### DESCRIPTION OF THE PREFERRED EMBODIMENTS

### [First Embodiment]

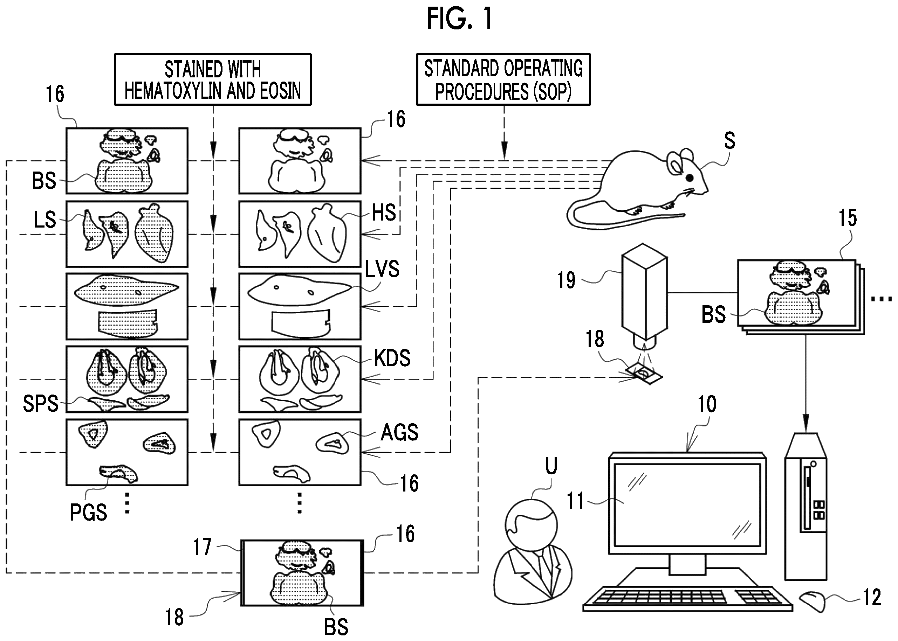

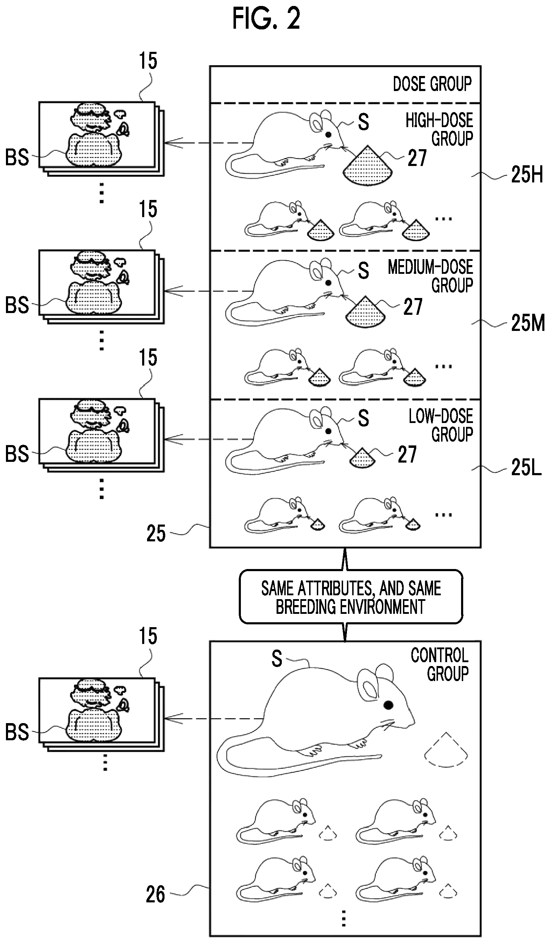

As shown in FIG. 1 as an example, an evaluation support apparatus 10 is used for evaluating drug efficacy and toxicity of a candidate substance 27 (see FIG. 2) of a drug. The evaluation support apparatus 10 is an example of an "image display device" according to the technology of the present disclosure. The drug is, for example, a biopharmaceutical such as an antibody pharmaceutical having an antibody as an active ingredient, or a peptide pharmaceutical, a nucleic acid pharmaceutical, or the like having a peptide or a nucleic acid as an active ingredient.

The evaluation support apparatus 10 is, for example, a desktop personal computer and comprises a display 11 that displays various screens and an input device 12 such as a keyboard, a mouse, a touch panel, and/or a microphone for voice input. The display 11 is an example of a "display unit" according to the technology of the present disclosure. The evaluation support apparatus 10 is installed in, for example, a pharmaceutical company that develops a drug or an institution that receives a development business of the drug from the pharmaceutical company, that is, a contract research organization (CRO). The evaluation support apparatus 10 is operated by a user U who is involved in the development of the drug in a pharmaceutical company or a contract research organization (hereinafter, collectively referred to as a pharmaceutical facility). The user U is, for example, a pathologist who is responsible for evaluating the candidate substance 27.

A plurality of specimen images 15 are input to the evaluation support apparatus 10. The specimen image 15 is an image for evaluating the drug efficacy and the toxicity of the candidate substance 27. The specimen image 15 is generated, for example, by the following procedure. First, a subject S such as a rat prepared for the evaluation of the candidate substance 27 is autopsied, and a tissue specimen obtained by slicing an organ of the subject S is collected. The tissue specimen includes a brain specimen BS, a heart specimen HS, a lung specimen LS, a liver specimen LVS, a kidney specimen KDS, a spleen specimen SPS, an adrenal gland specimen AGS, a pituitary gland specimen PGS, and the like. Although not shown in FIG. 1, the tissue specimen also includes specimens of various organs such as an esophagus, a stomach, a large intestine, a small intestine, a pancreas, a gallbladder, an aorta, a large vein, a lymph node, a trachea, a bronchus, a diaphragm, a pineal gland, a testis or an ovary, and a spinal cord. That is, the organs serving as sources of the tissue specimens are of a plurality of types.

After the collection of the tissue specimen, each tissue specimen is attached to a slide glass 16 in accordance with standard operating procedures (SOP) predetermined for each pharmaceutical facility. The standard operating procedures describe, for example, designation of a fine layout of the tissue specimen, such as attaching the heart specimen HS and the lung specimen LS to the same slide glass 16 side by side. In this way, a plurality of tissue specimens are attached to one slide glass 16.

Thereafter, the tissue specimen is stained, here stained with hematoxylin and eosin dye. Subsequently, the stained tissue specimen is covered with a cover glass 17 to complete a slide specimen 18. Then, the slide specimen 18 is set in an imaging apparatus 19, such as a digital optical microscope, and the specimen image 15 is captured by the imaging apparatus 19. In the specimen image 15 obtained in this way, the entire tissue specimen attached to the slide glass 16 is imaged. In other words, a plurality of tissue specimens are imaged in one specimen image 15. The specimen image 15 is referred to as a whole slide image (WSI). The specimen image 15 is assigned with a subject identification data (ID) for uniquely identifying the subject S, a specimen image ID for uniquely identifying the specimen image 15, an imaging date and time, and the like. The tissue specimen is also referred to as a tissue section. In addition, the staining may be staining with a hematoxylin dye alone, staining with a nuclear fast red dye, or the like.

As shown in FIG. 2 as an example, the subject S is divided into a dose group 25 and a control group 26. The dose group 25 is composed of a plurality of subjects S to which the candidate substance 27 is administered. The dose group 25 is further divided into a high-dose group 25H, a medium-dose group 25M, and a low-dose group 25L according to the dose of the candidate substance 27. By dividing the dose group 25 into the high-dose group 25H, the medium-dose group 25M, and the low-dose group 25L in this way, it is possible to determine the influence on the subject S according to the dose of the candidate substance 27. The dose group 25 is not limited to being divided into the three groups of the high-dose group 25H, the medium-dose group 25M, and the low-dose group 25L illustrated as an example, and the dose group 25 may be divided into two groups of the high-dose group 25H and the low-dose group 25L, or the dose group 25 may be divided into four or more groups.

The control group 26 is composed of a plurality of subjects S to which the candidate substance 27 is not administered, unlike the dose group 25. The number of subjects S constituting each of the high-dose group 25H, the medium-dose group 25M, and the low-dose group 25L and the number of subjects S constituting the control group 26 are the same, for example, about 5 to 10. The subject S constituting each of the high-dose group 25H, the medium-dose group 25M, and the low-dose group 25L and the subject S constituting the control group 26 are subjects S having the same attributes and placed in the same breeding environment. The same attributes include, for example, the same weekly age and/or the same gender. In addition, the same attributes also include the same weekly age composition ratio and/or the same gender composition ratio (for example, five males and five females). The same breeding environment means, for example, that feed is the same, that the temperature and humidity of a breeding space are the same, and/or that the size of the breeding space is the same. The "same" in the same breeding environment indicates not only the exact same, but also the same including an error that is generally allowed in the technical field to which the technology of the present disclosure belongs and that does not go against the gist of the technology of the present disclosure.

Since a plurality of specimen images 15 are obtained from one subject S, the number of specimen images 15 obtained from each group is obtained by multiplying the number of specimen images 15 obtained from one subject S by the number of subjects S. For example, in a case where the number of specimen images 15 obtained from one subject S is 100 and the number of subjects S constituting each group is 10, 100 × 10 = 1000 specimen images 15 are obtained from each group.

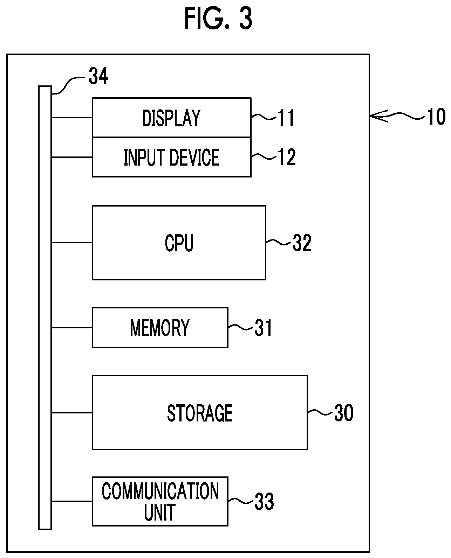

As shown in FIG. 3 as an example, a computer constituting the evaluation support apparatus 10 comprises a storage 30, a memory 31, a central processing unit (CPU) 32, and a communication unit 33 in addition to the display 11 and the input device 12 described above. These are connected to each other via a busline 34.

The storage 30 is a hard disk drive that is built into the computer constituting the evaluation support apparatus 10 or that is connected via a cable or a network. Alternatively, the storage 30 is a disk array in which a plurality of hard disk drives are connected in series. The storage 30 stores a control program, such as an operating system, various application programs, various types of data associated with these programs, and the like. A solid state drive may be used instead of the hard disk drive.

The memory 31 is a work memory for the CPU 32 to execute processing. The CPU 32 loads the program stored in the storage 30 into the memory 31 and executes the processing in accordance with the program. Therefore, the CPU 32 comprehensively controls each unit of the computer. In addition, the CPU 32 is an example of a "processor" according to the technology of the present disclosure. The memory 31 may be built into the CPU 32. The communication unit 33 controls the transmission of various types of information to an external device such as the imaging apparatus 19.

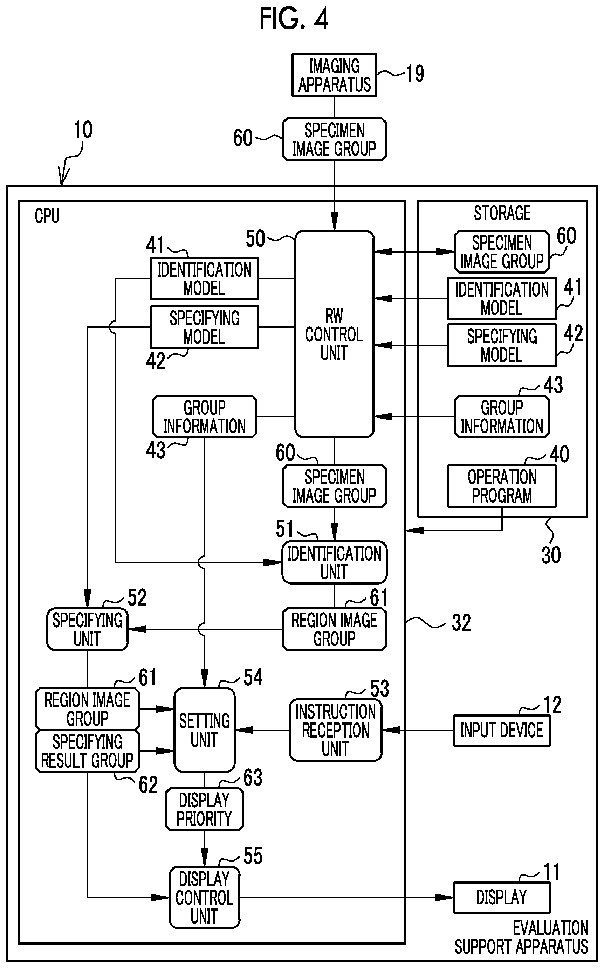

As shown in FIG. 4 as an example, an operation program 40 is stored in the storage 30 of the evaluation support apparatus 10. The operation program 40 is an application program for causing the computer to function as the evaluation support apparatus 10. That is, the operation program 40 is an example of an "operation program of an image display device" according to the technology of the present disclosure. The storage 30 also stores an identification model 41, a specifying model 42, group information 43, and the like.

In a case where the operation program 40 is activated, the CPU 32 of the computer constituting the evaluation support apparatus 10 functions as a read/write (hereinafter, abbreviated as RW) control unit 50, an identification unit 51, a specifying unit 52, an instruction reception unit 53, a setting unit 54, and a display control unit 55 in cooperation with the memory 31 and the like.

The RW control unit 50 controls the storage of various types of data in the storage 30 and the reading-out of various types of data in the storage 30. For example, the RW control unit 50 acquires a specimen image group 60 from the imaging apparatus 19 and stores the specimen image group 60 in the storage 30. The specimen image group 60 is a set of a plurality of specimen images 15 generated for evaluating the candidate substance 27.

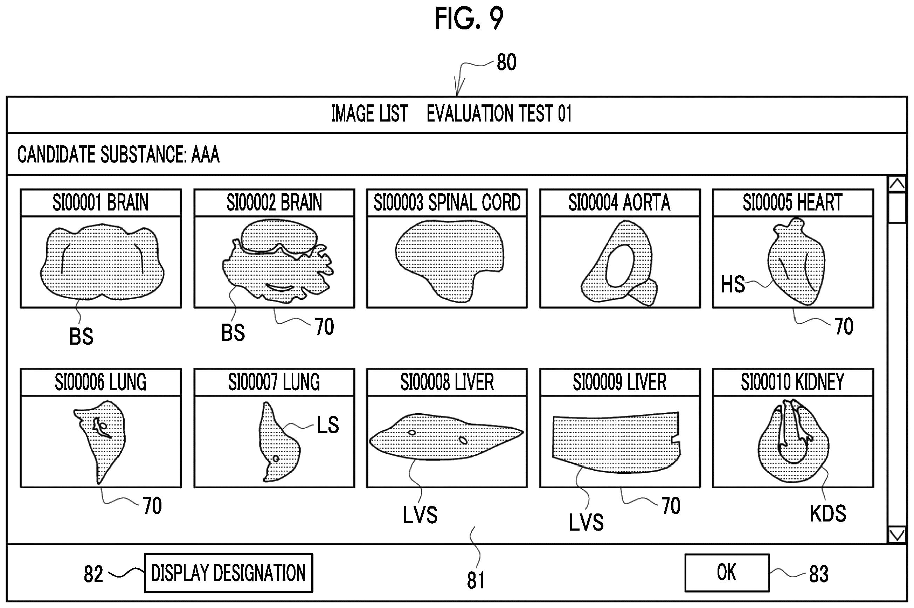

In a case where a display instruction of an image list display screen 80 (see FIG. 9) is issued by the user U through the input device 12, the RW control unit 50 reads out the specimen image group 60 from the storage 30. The RW control unit 50 outputs the read-out specimen image group 60 to the identification unit 51.

The RW control unit 50 reads out the identification model 41 from the storage 30 and outputs the read-out identification model 41 to the identification unit 51. In addition, the RW control unit 50 reads out the specifying model 42 from the storage 30 and outputs the read-out specifying model 42 to the specifying unit 52. Further, the RW control unit 50 reads out the group information 43 from the storage 30 and outputs the read-out group information 43 to the setting unit 54.

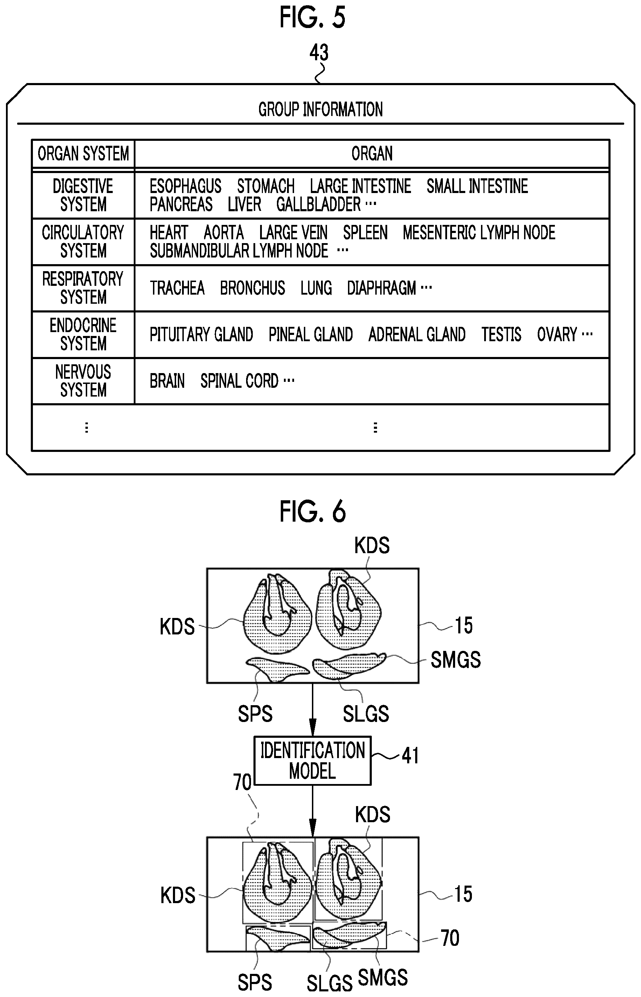

The identification unit 51 identifies the plurality of tissue specimens imaged in one specimen image 15 by using the identification model 41. Then, region images 70 (see FIG. 6) of the identified tissue specimens are generated from the specimen image 15. The identification unit 51 outputs a region image group 61, which is a set of the generated plurality of region images 70, to the specifying unit 52.

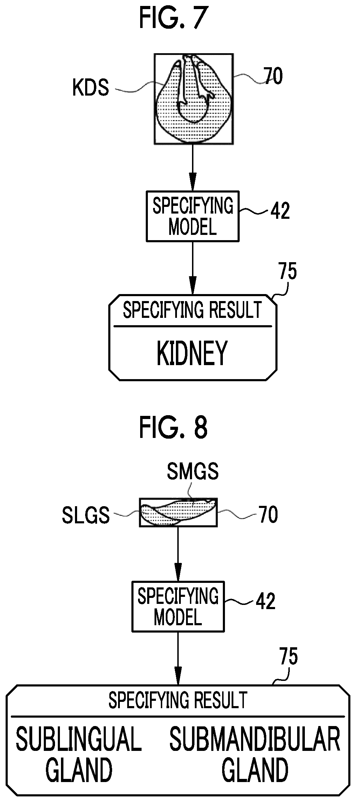

The specifying unit 52 specifies the types of the organs of the tissue specimens imaged in the region images 70 by using the specifying model 42. Then, a specifying result group 62, which is a set of specifying results 75 (see FIGS. 7 and 8) of the type of the organ, is output to the setting unit 54 and the display control unit 55 together with the region image group 61.

The instruction reception unit 53 receives various operation instructions from the user U through the input device 12. The various operation instructions include the display instruction of the image list display screen 80 and a display designation instruction of the region image 70.

In a case where the display designation instruction of the region image 70 is issued by the user U through the input device 12, the setting unit 54 sets a display priority 63 of the region image 70 based on the group information 43 and the specifying result group 62. The setting unit 54 outputs the set display priority 63 to the display control unit 55.

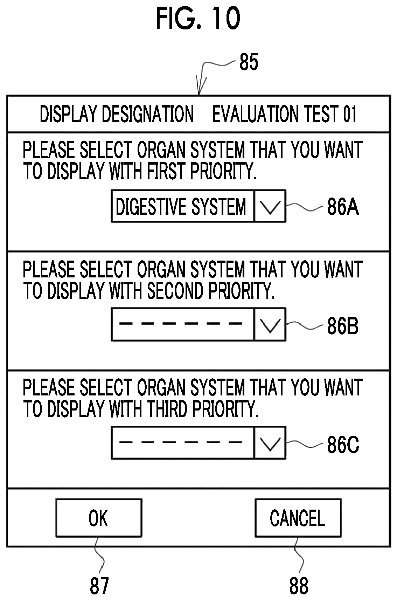

The display control unit 55 performs control of displaying various screens on the display 11. Various screens include the image list display screen 80 on which the region image 70 is displayed in a list, and a display designation screen 85 (see FIG. 10) for issuing the display designation instruction of the region image 70.

As shown in FIG. 5 as an example, the group information 43 is information in which a plurality of types of organs are classified into groups for each organ system. The organ system includes a digestive system, a circulatory system, a respiratory system, an endocrine system, a nervous system, and the like. For example, the organs constituting the group of the digestive system are an esophagus, a stomach, a large intestine, a small intestine, a pancreas, a liver, a gallbladder, and the like. In addition, the organs constituting the group of the endocrine system are a pituitary gland, a pineal gland, an adrenal gland, a testis or an ovary, and the like. In addition to these, the organ system includes a urinary system, a skeletal system, a muscular system, and the like.

As shown in FIG. 6 as an example, the identification unit 51 inputs the specimen image 15 to the identification model 41. Then, the identification model 41 identifies the plurality of tissue specimens imaged in the specimen image 15. The identification model 41 is, for example, a machine learning model such as a convolutional neural network. The identification model 41 identifies each of the plurality of tissue specimens imaged in the specimen image 15 one by one and outputs position coordinates of a rectangular frame (referred to as a bounding box) surrounding the tissue specimen as an identification result. The identification unit 51 generates the region image 70 of each identified tissue specimen by cutting out the rectangular frame from the specimen image 15 according to the identification result output by the identification model 41. A region image ID for uniquely identifying the region image 70 is assigned to the region image 70, as in the specimen image 15.

FIG. 6 illustrates the specimen image 15 in which two kidney specimens KDS, a spleen specimen SPS, a sublingual gland specimen SLGS, and a submandibular gland specimen SMGS are imaged. As can be seen from the example of the sublingual gland specimen SLGS and the submandibular gland specimen SMGS, a plurality of tissue specimens of organs may be mixed in the region image 70.

The rectangular frame surrounding the tissue specimen, which is the identification result of the identification model 41, may be configured to be modifiable by the user U. In addition, the tissue specimen imaged in the specimen image 15 may be identified by template matching instead of the identification model 41. Alternatively, the tissue specimen imaged in the specimen image 15 may be identified by inputting the rectangular frame surrounding the tissue specimen by hand of the user U without using the identification model 41 or the template matching.

As shown in FIGS. 7 and 8 as an example, the specifying unit 52 inputs the region image 70 to the specifying model 42. Then, the specifying model 42 specifies the types of the organs of the tissue specimens imaged in the region images 70. The specifying model 42 is also, for example, a machine learning model such as a convolutional neural network, as in the identification model 41. The specifying model 42 outputs the specifying result 75 of the type of the organ of the tissue specimen imaged in the region image 70.

FIG. 7 illustrates a case where the region image 70 in which the kidney specimen KDS is imaged is input to the specifying model 42, and the specifying result 75 of "kidney" is output from the specifying model 42. In addition, FIG. 8 illustrates a case where the region image 70 in which the sublingual gland specimen SLGS and the submandibular gland specimen SMGS are imaged is input to the specifying model 42, and the specifying result 75 of "sublingual gland and submandibular gland" is output from the specifying model 42.

The specifying result 75 may be configured to be modifiable by the user U, or the specifying of the type of the organ of the tissue specimen imaged in the region image 70 may be entrusted to the hand of the user U. In addition, the types of the organs of the tissue specimens imaged in the region images 70 may be specified by template matching instead of the specifying model 42.

The display control unit 55 performs control of displaying, on the display 11, the image list display screen 80 shown in FIG. 9 as an example in response to the display instruction from the user U through the input device 12. The image list display screen 80 includes a display region 81. All the region images 70 generated by the identification unit 51 are displayed in a list in the display region 81. In the display region 81, the region images 70 are arranged in order of the region image ID from top to bottom and from left to right. An organ name based on the specifying result 75 is displayed in the region image 70 together with the region image ID. Each region image 70 can be selected and enlarged and displayed, and the user U can observe each region image 70 in detail. In a case where the enlarged region image 70 is obtained from the subject S belonging to the dose group 25, the region image 70 of the same organ obtained from the subject S of the control group 26 may be displayed side by side for comparison. Alternatively, in the image list display screen 80, the region image 70 obtained from the subject S belonging to the dose group 25 and the region image 70 obtained from the subject S belonging to the control group 26 may be displayed side by side.

A display designation button 82 and an OK button 83 are provided below the display region 81. In a case where the display designation button 82 is selected, the display control unit 55 performs control of displaying, on the display 11, a display designation screen 85 shown in FIG. 10 as an example. On the other hand, in a case where the OK button 83 is selected, the display control unit 55 erases the display of the image list display screen 80.

In FIG. 10, the display designation screen 85 is provided with pull-down menus 86A, 86B, and 86C. The pull-down menu 86A is for the user U to select the organ system that the user U wants to display with first priority. The pull-down menu 86B is for the user U to select the organ system that the user U wants to display with second priority. The pull-down menu 86C is for the user U to select the organ system that the user U wants to display with third priority. Selection of the pull-down menus 86B and 86C is not essential. In addition, the organ system selected in one of the pull-down menus 86A to 86C cannot be selected in a pull-down menu other than one of the pull-down menus 86A to 86C. As described above, the display designation screen 85 is a screen for receiving designation of a group based on the organ system, which is a criterion for setting the display priority 63 of the plurality of region images 70.

In a case where the user U selects a desired organ system in at least the pull-down menu 86A and then the OK button 87 is selected, the instruction reception unit 53 receives the display designation instruction for setting the group of the organ system selected in the pull-down menu 86A or the like as the criterion for setting the display priority 63 of the region image 70. On the other hand, in a case where a cancel button 88 is selected, the display control unit 55 erases the display of the display designation screen 85. FIG. 10 illustrates a case where the digestive system is selected in the pull-down menu 86A.

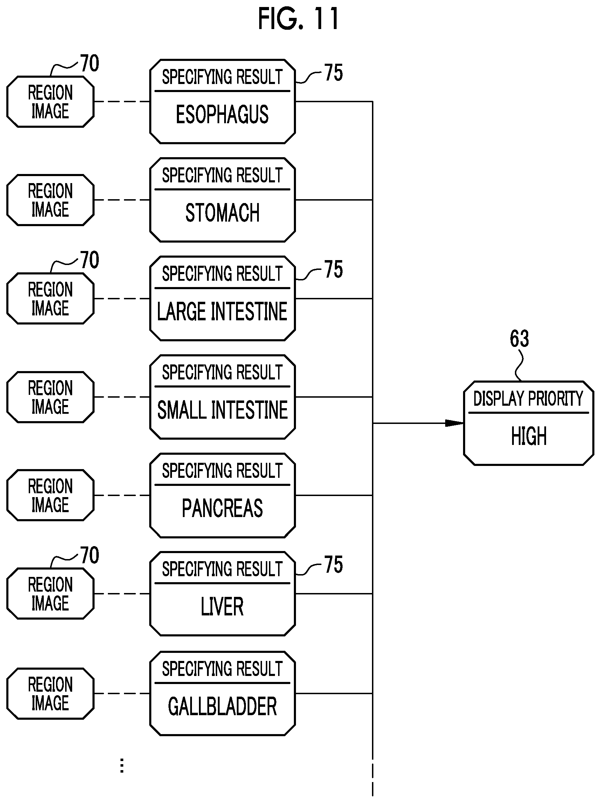

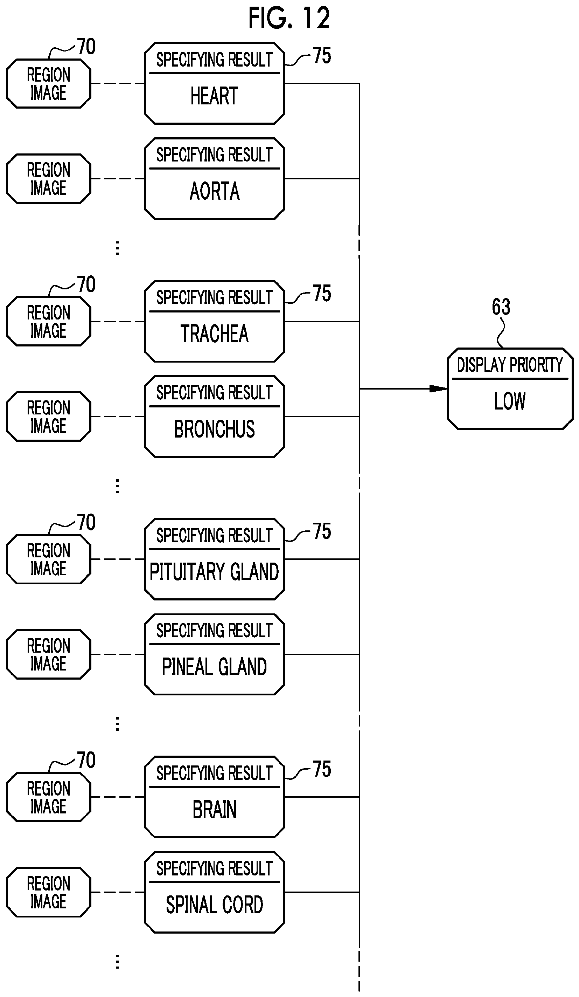

In a case where the display designation instruction is received by the instruction reception unit 53, the setting unit 54 sets the display priority 63 corresponding to the display designation instruction. Specifically, as shown in FIG. 11 as an example, the setting unit 54 sets the display priority 63 of the region images 70 in which the tissue specimens of the organs belonging to the group of the organ system that have received the display designation instruction are imaged to "high". On the other hand, as shown in FIG. 12 as an example, the setting unit 54 sets the display priority 63 of the region images 70 in which the tissue specimens of the organs belonging to the group other than the group of the organ system that have received the display designation instruction are imaged to "low". That is, the setting unit 54 sets the display priority 63 of the region images 70 in which the tissue specimens of the organs belonging to the group of the organ system that have received the display designation instruction are imaged to be higher than the display priority 63 of the region images 70 in which the tissue specimens of the organs belonging to the group other than the group of the organ system that have received the display designation instruction are imaged.

FIGS. 11 and 12 illustrate a case where the digestive system is selected in the pull-down menu 86A shown in FIG. 10. Therefore, the setting unit 54 sets the display priority 63 of the region image 70, which is the specifying result 75 of the esophagus, the stomach, the large intestine, the small intestine, the pancreas, the liver, the gallbladder, and the like belonging to the digestive system, to "high". On the other hand, the display priority 63 of the region image 70, which is the specifying result 75 of the heart, the trachea, the pituitary gland, the brain, and the like belonging to the organ system other than the digestive system, is set to "low".

In a case where two organ systems are selected in the pull-down menus 86A and 86B, the setting unit 54 sets the display priority 63 of the region images 70 in which the tissue specimens of the organs belonging to the group of the organ system selected in the pull-down menu 86A are imaged to "high". In addition, the display priority 63 of the region images 70 in which the tissue specimens of the organs belonging to the group of the organ system selected in the pull-down menu 86B are imaged is set to "medium". Further, the display priority 63 of the region images 70 in which the tissue specimens of the organs belonging to the group of the organ system are imaged other than these is set to "low".

In addition, in a case where three organ systems are selected in the pull-down menus 86A to 86C, the setting unit 54 sets the display priority 63 of the region images 70 in which the tissue specimens of the organs belonging to the group of the organ system selected in the pull-down menu 86A are imaged to "high". In addition, the display priority 63 of the region images 70 in which the tissue specimens of the organs belonging to the group of the organ system selected in the pull-down menu 86B are imaged is set to "medium high", and the display priority 63 of the region images 70 in which the tissue specimens of the organs belonging to the group of the organ system selected in the pull-down menu 86C are imaged is set to "medium low". Further, the display priority 63 of the region images 70 in which the tissue specimens of the organs belonging to the group of the organ system are imaged other than these is set to "low".

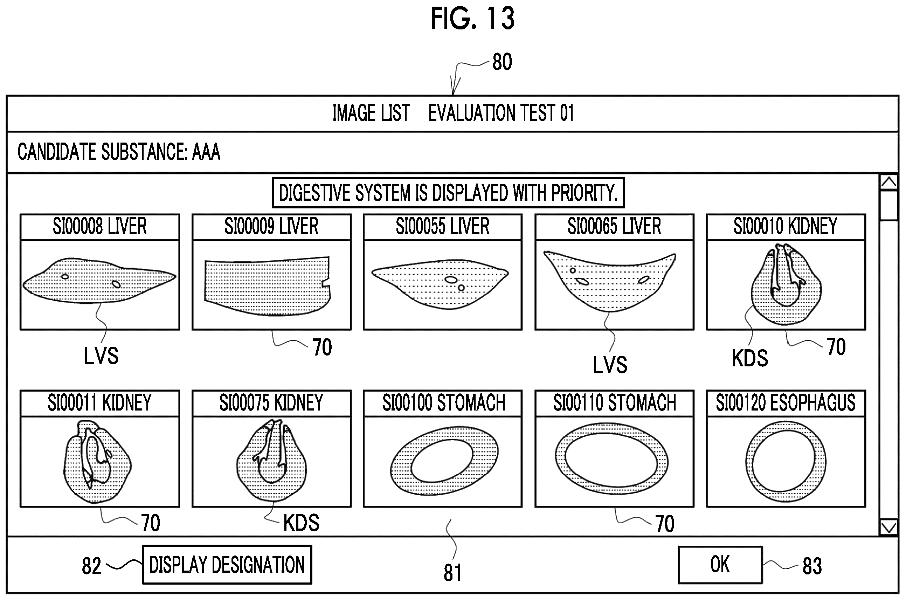

As shown in FIG. 13 as an example, the display control unit 55 receives the display priority 63 from the setting unit 54 and updates the display of the image list display screen 80. Specifically, in the display region 81, the display order of the region images 70 is rearranged in descending order of the display priority 63. FIG. 13 illustrates a case where the digestive system is selected in the pull-down menu 86A shown in FIGS. 10 to 12. Therefore, the region image 70 in which each tissue specimen of the esophagus, the stomach, the large intestine, the small intestine, the pancreas, the liver, the gallbladder, and the like belonging to the digestive system is imaged is displayed with priority at the upper part of the display region 81.

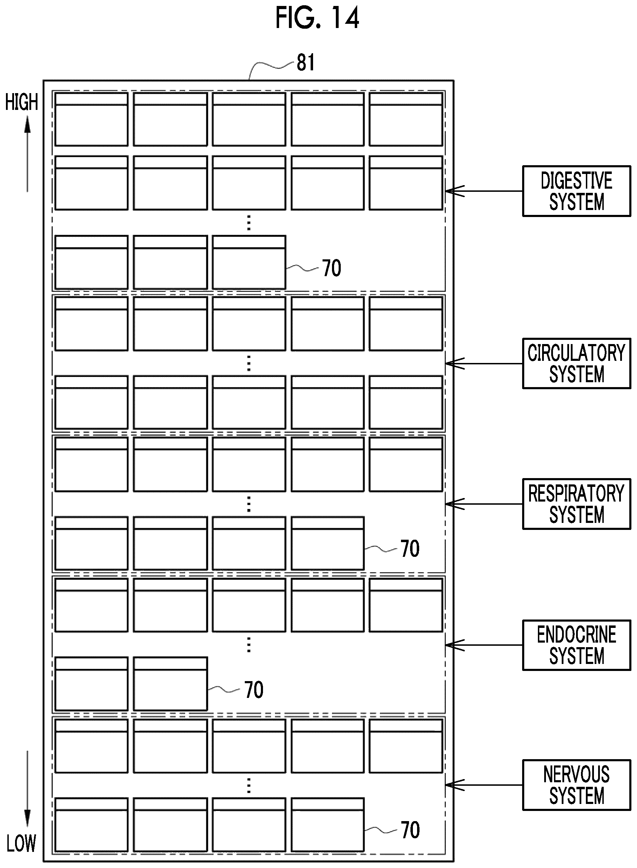

As shown in FIG. 14 as an example, the display control unit 55 performs control of displaying the region images 70 collectively for each group of each organ system such as the digestive system, the circulatory system, the respiratory system, the endocrine system, and the nervous system in the display region 81. FIG. 14 illustrates a case where the digestive system is selected in the pull-down menu 86A shown in FIGS. 10 to 12.

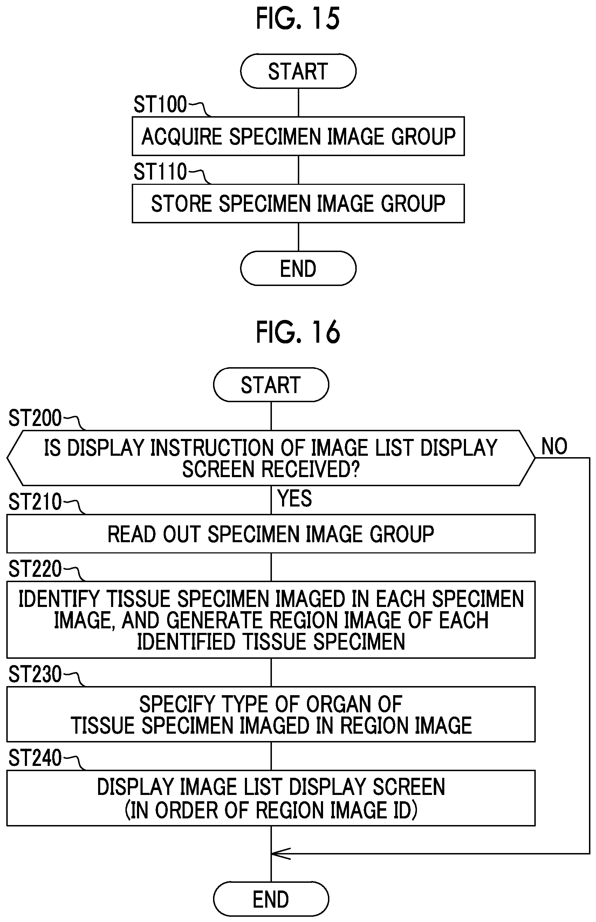

Next, an operation of the configuration described above will be described with reference to the flowchart shown in FIGS. 15 to 17 as an example. First, in a case where the operation program 40 is activated in the evaluation support apparatus 10, as shown in FIG. 4, the CPU 32 of the evaluation support apparatus 10 functions as the RW control unit 50, the identification unit 51, the specifying unit 52, the instruction reception unit 53, the setting unit 54, and the display control unit 55.

The imaging apparatus 19 captures the specimen image 15 of the tissue specimen of the subject S. The specimen image 15 is transmitted from the imaging apparatus 19 to the evaluation support apparatus 10. In the evaluation support apparatus 10, as shown in FIG. 15, the specimen image group 60, which is a set of the specimen images 15 from the imaging apparatus 19, is acquired by the RW control unit 50 (step ST100). The specimen image group 60 is stored in the storage 30 under the control of the RW control unit 50 (step ST110).

In FIG. 16, in a case where the display instruction of the image list display screen 80 is issued by the user U through the input device 12 and the display instruction is received by the instruction reception unit 53 (YES in step ST200), the specimen image group 60 designated by the display instruction is read out from the storage 30 by the RW control unit 50 (step ST210). The specimen image group 60 is output from the RW control unit 50 to the identification unit 51.

In the identification unit 51, as shown in FIG. 6, the specimen image 15 is input to the identification model 41, and the plurality of tissue specimens imaged in the specimen image 15 are identified, and the identification result is output from the identification model 41. Then, the region image 70 of each tissue specimen is generated based on the identification result (step ST220). The region image group 61, which is a set of the region images 70, is output from the identification unit 51 to the specifying unit 52.

In the specifying unit 52, as shown in FIGS. 7 and 8, the region image 70 is input to the specifying model 42. As a result, the type of the organ of the tissue specimen imaged in the region image 70 is specified (step ST230), and the specifying result 75 is output from the specifying model 42. The specifying result group 62, which is a set of the specifying results 75, is output to the setting unit 54 and the display control unit 55 together with the region image group 61. As shown in FIG. 9, the image list display screen 80 in which the region images 70 are arranged in the display region 81 in the order of the region image ID is displayed on the display 11 under the control of the display control unit 55 (step ST240).

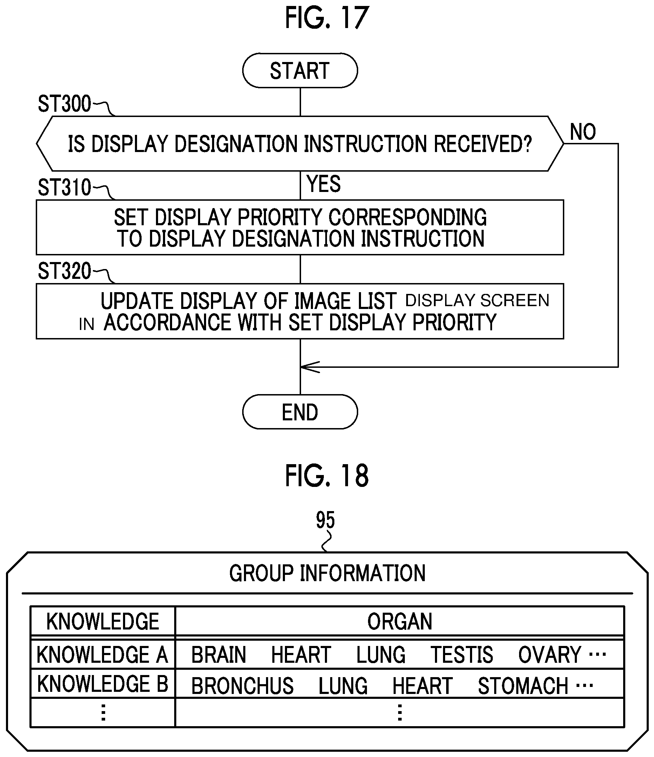

The user U selects the display designation button 82 to rearrange the display of the region image 70 from the viewpoint desired by the user U. As a result, the display designation screen 85 shown in FIG. 10 is displayed on the display 11 under the control of the display control unit 55. The user U selects a desired organ system in the pull-down menus 86A to 86C and selects the OK button 87. As a result, as shown in FIG. 17, the display designation instruction including the organ system selected in the pull-down menu 86A or the like is received by the instruction reception unit 53 (YES in step ST300).

In the setting unit 54, as shown in FIGS. 11 and 12, the display priority 63 corresponding to the display designation instruction is set (step ST310). More specifically, the display priority 63 of the region images 70 in which the tissue specimens of the organs belonging to the group of the organ system that have received the display designation instruction are imaged is set to be higher than the display priority 63 of the region images 70 in which the tissue specimens of the organs belonging to the group other than the group of the organ system that have received the display designation instruction are imaged. The display priority 63 is output from the setting unit 54 to the display control unit 55.

As shown in FIG. 13, the display control unit 55 that has received the display priority 63 updates the display of the image list display screen 80 in accordance with the display priority 63 (step ST320). The user U observes the region image 70 on the image list display screen 80 on which the display is updated, and evaluates the drug efficacy and the toxicity of the candidate substance 27.

As described above, the CPU 32 of the evaluation support apparatus 10 comprises the RW control unit 50, the instruction reception unit 53, the setting unit 54, and the display control unit 55. The RW control unit 50 acquires a plurality of specimen images 15 in which tissue specimens of the subject S provided for the evaluation test of the candidate substance 27 of the drug are imaged. The instruction reception unit 53 receives the display designation instruction of the group of the organ system, which is the criterion for setting the display priority 63 of the plurality of region images 70 generated from the plurality of specimen images 15, by the user U. The setting unit 54 sets the display priority 63 corresponding to the display designation instruction. The display control unit 55 performs control of displaying the plurality of region images 70 on the display 11 in accordance with the set display priority 63. Therefore, it is possible to set the display priority 63 of the region image 70 in accordance with the viewpoint desired by the user U. As a result, it is possible to greatly contribute to the improvement of the efficiency of the evaluation of the drug efficacy and the toxicity of the candidate substance 27 of the drug and the improvement of the accuracy of the evaluation.

As shown in FIG. 1, the organs serving as sources of the tissue specimens are of a plurality of types. As shown in FIG. 5, the plurality of types of organs are classified into a plurality of groups in advance. As shown in FIG. 10, the instruction reception unit 53 receives the designation of the group as the designation of the criterion. The specifying unit 52 specifies the types of the organs of the tissue specimens imaged in the region images 70. The setting unit 54 sets the display priority 63 of the region images 70 in which the tissue specimens of the organs belonging to the group that have received the display designation instruction are imaged to be higher than the display priority 63 of the region images 70 in which the tissue specimens of the organs belonging to the group other than the group that have received the display designation instruction are imaged. Therefore, the region images 70 in which the tissue specimens of the organs belonging to the group that the user U wants to observe with priority are imaged can be provided for the browsing by the user U with priority.

As shown in FIG. 14, the display control unit 55 performs control of displaying the region images 70 collectively for each group. Therefore, the region images 70 can be observed in an organized manner as compared with a case where the region images 70 are displayed in a disorderly manner without the constraint of the group. It is possible to further contribute to the improvement of the efficiency of the evaluation of the drug efficacy and the toxicity of the candidate substance 27 of the drug and the improvement of the accuracy of the evaluation.

As shown in FIG. 1, a plurality of tissue specimens are imaged in one specimen image 15. The identification unit 51 identifies the plurality of tissue specimens imaged in the one specimen image 15. The specifying unit 52 specifies the types of the organs of the identified tissue specimens. As illustrated above, the slide specimen 18 generally includes a plurality of tissue specimens, and it is common that a plurality of tissue specimens are imaged in one specimen image 15. Therefore, the above-described processing can be regarded as processing that is in line with general operation.

As shown in FIG. 6, the identification unit 51 generates the region image 70 of the identified tissue specimen from the specimen image 15. As shown in FIG. 13, the display control unit 55 rearranges the region images 70 in accordance with the set display priority 63.

The type, the number, and the like of the organs of the tissue specimen attached to the slide glass 16 are designated in advance in the standard operating procedures. The layout of the organ designated in the standard operating procedures does not necessarily consider the organ system. In addition, the standard operating procedures cannot be easily changed due to the reason that the consent of all the users U of the pharmaceutical facility is required. Therefore, in some cases, the specimen image 15 in which the tissue specimens of the organs of various organ systems are mixed is obtained as the specimen image 15, and even in a case where the display priority 63 is set in units of the group of the organ system, the specimen image 15 cannot be rearranged in accordance with the display priority 63. On the other hand, since the region image 70 is an image in which each tissue specimen imaged in the specimen image 15 is cut out one by one, the region image 70 can be rearranged in accordance with the display priority 63 set in units of the group of the organ system. The display order of the region images 70 can be freely rearranged without performing an extremely troublesome procedure of obtaining the consent of all the users U in order to change the standard operating procedures.

Organs belonging to the same organ system have common functions and work together. Therefore, the organs belonging to the same organ system are likely to commonly develop changes in response to the drug efficacy and the toxicity of the candidate substance 27 of the drug, particularly morphological abnormalities caused by the toxicity. Therefore, as in the present embodiment, in a case where the group designated by the display designation instruction is set to the group based on the organ system, the probability of being able to collectively observe the region image 70 in which the tissue specimen of the organ in which the morphological abnormality has occurred is imaged with priority is increased. It is possible to further contribute to the improvement of the efficiency of the evaluation of the drug efficacy and the toxicity of the candidate substance 27 of the drug and the improvement of the accuracy of the evaluation. The morphological abnormality is a lesion that is not observed in a normal tissue specimen, for example, hyperplasia, infiltration, congestion, cyst, inflammation, tumor, carcinogenesis, proliferation, bleeding, or glycogen reduction.

The group is not limited to the group based on the organ system. As group information 95 shown in FIG. 18 as an example, the group may be a group based on the knowledge of the user U. The group information 95 is input in advance by the user U and stored in the storage 30. The knowledge of the user U is, for example, knowledge obtained in a case where a candidate substance similar to the candidate substance 27 currently being evaluated has been evaluated in the past, in a case where the morphological abnormality has occurred in an organ A, the morphological abnormality occurs in organs B and C at a high probability. The knowledge may be knowledge obtained for all the candidate substances evaluated in the past, not limited to the candidate substance similar to the candidate substance 27 currently being evaluated.

As described above, in a case where the group is set to the group based on the knowledge of the user U, the probability of being able to collectively observe the region image 70 in which the tissue specimen of the organ in which the morphological abnormality has occurred is imaged with priority is further increased. It is possible to further contribute to the improvement of the efficiency of the evaluation of the drug efficacy and the toxicity of the candidate substance 27 of the drug and the improvement of the accuracy of the evaluation.

In the image list display screen 80 in the initial state shown in FIG. 9, for example, a selection instruction of the region image 70 for enlarged display may be regarded as the display designation instruction. Specifically, the group of the organ system to which the organ imaged in the enlarged region image 70 belongs is received as the criterion for setting the display priority 63 of the region image 70. In this case, in a case where the enlarged display is released, the image list display screen 80 in which the display order of the region images 70 is rearranged in accordance with the display priority 63 is displayed.

### [Second Embodiment]

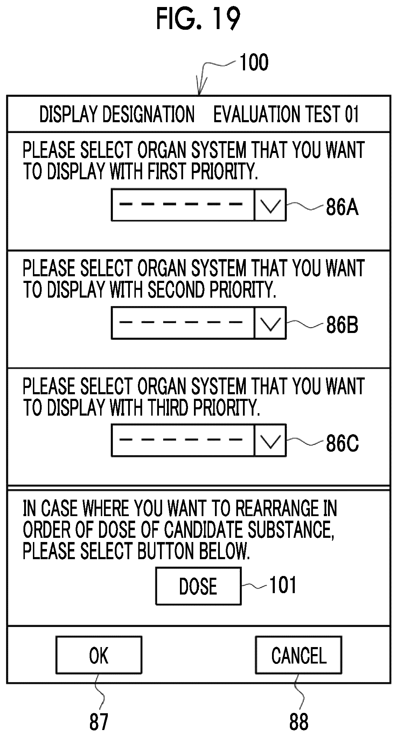

As shown in FIG. 19 as an example, a display designation screen 100 of a second embodiment is provided with a dose button 101 in addition to the pull-down menus 86A to 86C and the like. In a case where the dose button 101 is selected and the OK button 87 is selected by the user U, the instruction reception unit 53 receives the display designation instruction for setting the dose of the candidate substance 27 to the subject S as the criterion for setting the display priority 63 of the region image 70.

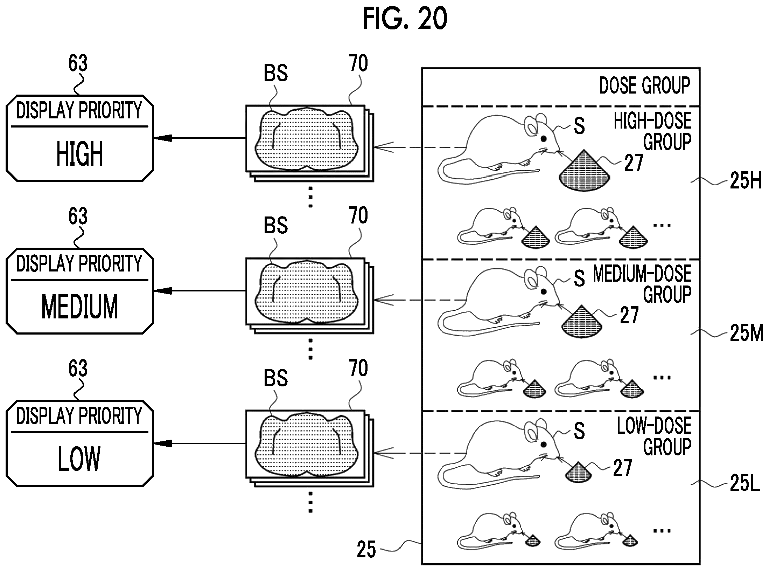

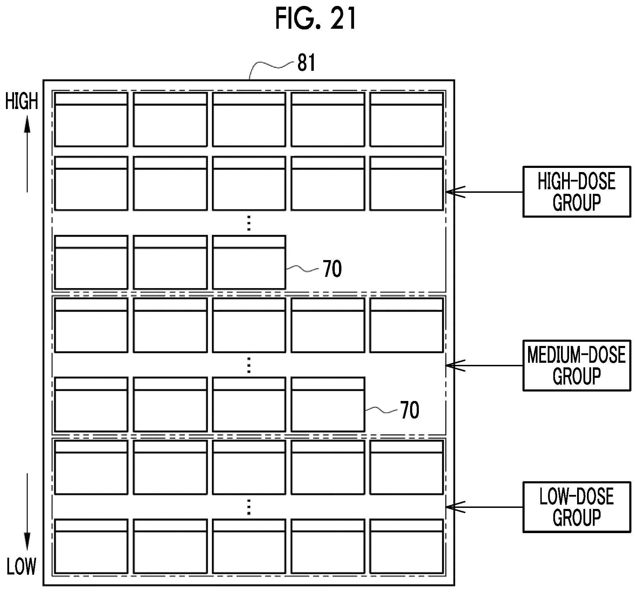

In a case where the display designation instruction is received by the instruction reception unit 53, as shown in FIG. 20 as an example, the setting unit 54 sets the display priority 63 of the region image 70 obtained from the subject S belonging to the high-dose group 25H to "high". In addition, the display priority 63 of the region image 70 obtained from the subject S belonging to the medium-dose group 25M is set to "medium". Further, the display priority 63 of the region image 70 obtained from the subject S belonging to the low-dose group 25L is set to "low". That is, the setting unit 54 sets the display priority 63 of the region images 70 in which the tissue specimens of the subject S having a relatively large dose of the candidate substance 27 are imaged to be higher than the display priority 63 of the region images 70 in which the tissue specimens of the subject S having a relatively small dose of the candidate substance 27 are imaged. Therefore, as shown in FIG. 21 as an example, the display control unit 55 performs control of displaying the region images 70 in the display region 81 in the order of the high-dose group 25H, the medium-dose group 25M, and the low-dose group 25L.

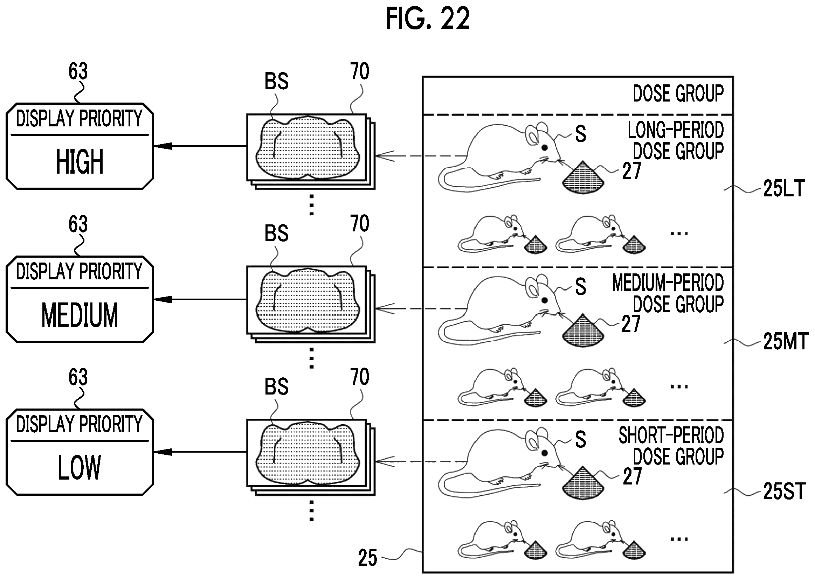

The division of the dose group 25 is not limited to the division according to the dose of the candidate substance 27 to the subject S illustrated as an example. As shown in FIG. 22 as an example, the dose group 25 may be divided according to the length of the dosing period of the candidate substance 27 to the subject S. FIG. 22 illustrates a case where the dose group 25 is divided into a long-period dose group 25LT, a medium-period dose group 25MT, and a short-period dose group 25ST. The long-period dose group 25LT is a set of subjects S to which the candidate substance 27 is administered for, for example, 6 months, the medium-period dose group 25MT is a set of subjects S to which the candidate substance 27 is administered for, for example, 4 months, and the short-period dose group 25ST is a set of subjects S to which the candidate substance 27 is administered for, for example, 2 months.

In this case, a dosing period button for performing the display designation instruction for setting the dosing period of the candidate substance 27 to the subject S as the criterion for setting the display priority 63 of the region image 70 is provided on the display designation screen. Then, in a case where the dosing period button is selected and the OK button 87 is selected and the display designation instruction is received by the instruction reception unit 53, the setting unit 54 sets the display priority 63 of the region image 70 obtained from the subject S belonging to the long-period dose group 25LT to "high". In addition, the display priority 63 of the region image 70 obtained from the subject S belonging to the medium-period dose group 25MT is set to "medium". Further, the display priority 63 of the region image 70 obtained from the subject S belonging to the short-period dose group 25ST is set to "low". That is, the setting unit 54 sets the display priority 63 of the region images 70 in which the tissue specimens of the subject S having a relatively long dosing period of the candidate substance 27 are imaged to be higher than the display priority 63 of the region images 70 in which the tissue specimens of the subject S having a relatively short dosing period of the candidate substance 27 are imaged. The dose group 25 is not limited to being divided into the three groups of the long-period dose group 25LT, the medium-period dose group 25MT, and the short-period dose group 25ST illustrated as an example, and the dose group 25 may be divided into two groups of the long-period dose group 25LT and the short-period dose group 25ST, or the dose group 25 may be divided into four or more groups.

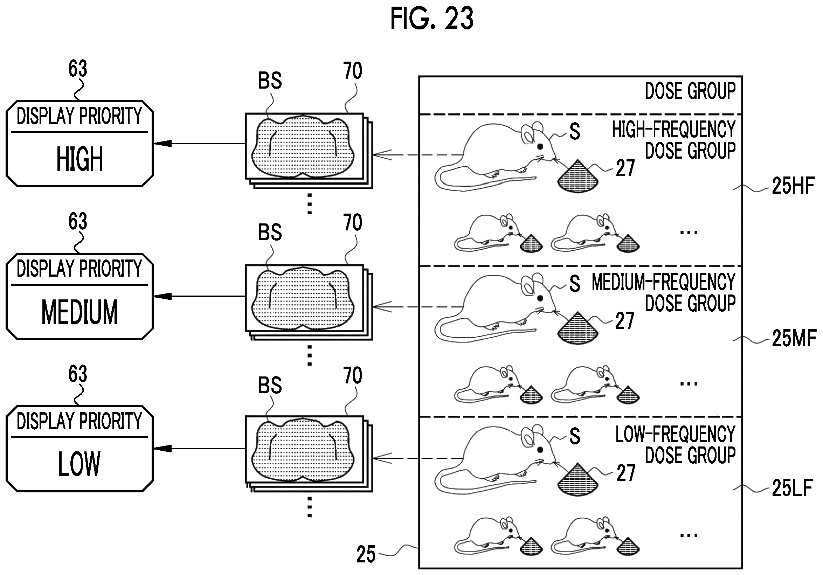

In addition, as shown in FIG. 23 as an example, the dose group 25 may be divided according to the dosing frequency of the candidate substance 27 to the subject S. FIG. 23 illustrates a case where the dose group 25 is divided into a high-frequency dose group 25HF, a medium-frequency dose group 25MF, and a low-frequency dose group 25LF. The high-frequency dose group 25HF is a set of subjects S to which the candidate substance 27 is administered, for example, three times a day, the medium-frequency dose group 25MF is a set of subjects S to which the candidate substance 27 is administered, for example, twice a day, and the low-frequency dose group 25LF is a set of subjects S to which the candidate substance 27 is administered, for example, once a day.

In this case, a dosing frequency button for performing the display designation instruction for setting the dosing frequency of the candidate substance 27 to the subject S as the criterion for setting the display priority 63 of the region image 70 is provided on the display designation screen. Then, in a case where the dosing frequency button is selected and the OK button 87 is selected and the display designation instruction is received by the instruction reception unit 53, the setting unit 54 sets the display priority 63 of the region image 70 obtained from the subject S belonging to the high-frequency dose group 25HF to "high". In addition, the display priority 63 of the region image 70 obtained from the subject S belonging to the medium-frequency dose group 25MF is set to "medium". Further, the display priority 63 of the region image 70 obtained from the subject S belonging to the low-frequency dose group 25LF is set to "low". That is, the setting unit 54 sets the display priority 63 of the region images 70 in which the tissue specimens of the subject S having a relatively high dosing frequency of the candidate substance 27 are imaged to be higher than the display priority 63 of the region images 70 in which the tissue specimens of the subject S having a relatively low dosing frequency of the candidate substance 27 are imaged. The dose group 25 is not limited to being divided into the three groups of the high-frequency dose group 25HF, the medium-frequency dose group 25MF, and the low-frequency dose group 25LF illustrated as an example, and the dose group 25 may be divided into two groups of the high-frequency dose group 25HF and the low-frequency dose group 25LF, or the dose group 25 may be divided into four or more groups.

As described above, in the second embodiment, the instruction reception unit 53 receives designation of any one of the dose, the dosing period, or the dosing frequency of the candidate substance 27 to the subject S as the designation of the criterion. The setting unit 54 sets the display priority of the region images 70 in which the tissue specimens of the subject S having a relatively large dose, a relatively long dosing period, or a relatively high dosing frequency are imaged to be higher than the display priority of the region images 70 in which the tissue specimens of the subject S having a relatively small dose, a relatively short dosing period, or a relatively low dosing frequency are imaged.

In general, the subject S having a relatively large dose, a relatively long dosing period, or a relatively high dosing frequency is likely to develop a morphological abnormality as compared with the subject S having a relatively small dose, a relatively short dosing period, or a relatively low dosing frequency. Therefore, according to the second embodiment, the probability of being able to collectively observe the region image 70 in which the tissue specimen of the organ in which the morphological abnormality has occurred is imaged with priority is increased. It is possible to contribute to the improvement of the efficiency of the evaluation of the drug efficacy and the toxicity of the candidate substance 27 of the drug and the improvement of the accuracy of the evaluation.

In FIG. 19, an example is shown in which the group of the organ system is not selected in the pull-down menus 86A to 86C, but the present disclosure is not limited to this. The dose button 101 may be selected after the group of the organ system is selected in the pull-down menus 86A to 86C. That is, the first embodiment and the second embodiment may be implemented in combination.

In a case where the dose button 101 is selected after the group of the organ system is selected in the pull-down menus 86A to 86C, the region images 70 are rearranged in the group of the organ system in descending order of the dose of the candidate substance 27 under the control of the display control unit 55. Similarly to the case of dividing the dose group 25 according to the length of the dosing period of the candidate substance 27 to the subject S shown in FIG. 22, in a case where the dosing period button is selected after the group of the organ system is selected in the pull-down menus 86A to 86C, the region images 70 are rearranged in the group of the organ system in descending order of the dosing period of the candidate substance 27 under the control of the display control unit 55. Similarly to the case of dividing the dose group 25 according to the dosing frequency of the candidate substance 27 to the subject S shown in FIG. 23, in a case where the dosing frequency button is selected after the group of the organ system is selected in the pull-down menus 86A to 86C, the region images 70 are rearranged in the group of the organ system in descending order of the dosing frequency of the candidate substance 27 under the control of the display control unit 55.

### [Third Embodiment]

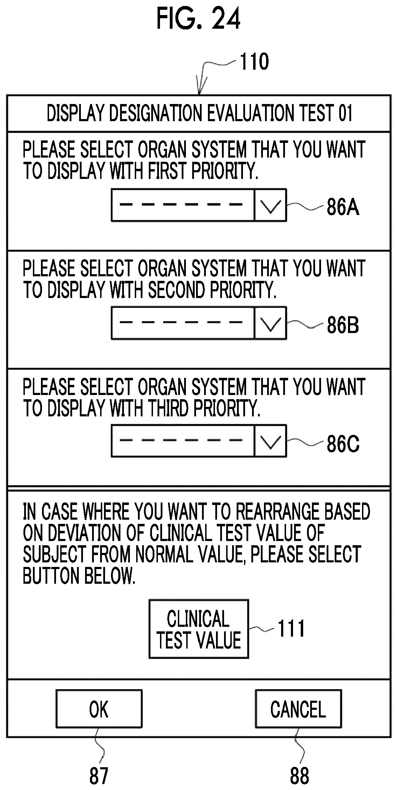

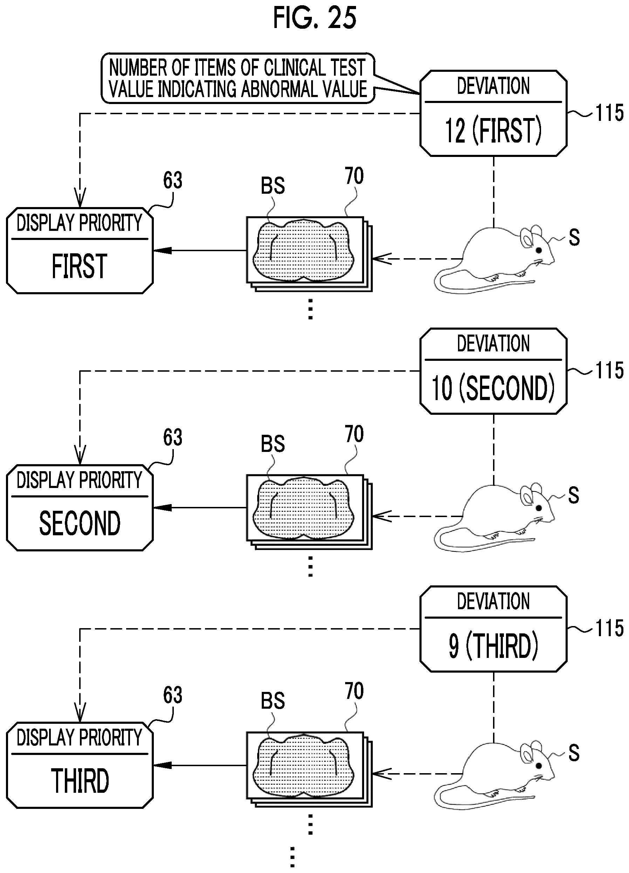

As shown in FIG. 24 as an example, a display designation screen 110 of a third embodiment is provided with a clinical test value button 111 in addition to the pull-down menus 86A to 86C and the like. In a case where the clinical test value button 111 is selected and the OK button 87 is selected by the user U, the instruction reception unit 53 receives the display designation instruction for setting a deviation 115 (see FIG. 25) of the clinical test value of the subject S from a normal value as the criterion for setting the display priority 63 of the region image 70. The clinical test value is a value measured after the administration of the candidate substance 27 is ended. The clinical test value is, for example, a blood test value, a urine test value, or the like, and has a plurality of items such as total protein, total cholesterol, red blood cell count, creatinine, uric acid, urine sugar, and urine specific gravity.

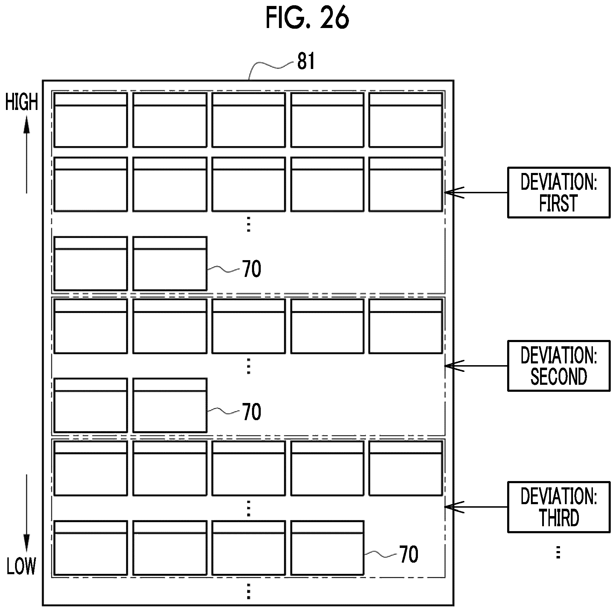

As shown in FIG. 25 as an example, the setting unit 54 derives the deviation 115 of the clinical test value of each subject S from the normal value. The deviation 115 is the number of items of the clinical test value indicating an abnormal value here. In a case where the display designation instruction is received by the instruction reception unit 53, the setting unit 54 sets the display priority 63 of the region image 70 obtained from the subject S having the first (maximum) deviation 115 to "first". In addition, the display priority 63 of the region image 70 obtained from the subject S having the second deviation 115 is set to "second". Further, the display priority 63 of the region image 70 obtained from the subject S having the third deviation 115 is set to "third". Similarly, the setting unit 54 sets the display priority 63 corresponding to the rank of the deviation 115. That is, the setting unit 54 sets the display priority 63 of the region images 70 in which the tissue specimens of the subject S having a relatively large deviation 115 of the clinical test value from the normal value are imaged to be higher than the display priority 63 of the region images 70 in which the tissue specimens of the subject S having a relatively small deviation 115 of the clinical test value from the normal value are imaged. Therefore, as shown in FIG. 26 as an example, the display control unit 55 performs control of displaying the region images 70 in the display region 81 in descending order of the deviation 115.

As described above, in the third embodiment, the instruction reception unit 53 receives the designation of the clinical test value of the subject S as the designation of the criterion. The setting unit 54 sets the display priority 63 of the region images 70 in which the tissue specimens of the subject S having a relatively large deviation 115 of the clinical test value from the normal value are imaged to be higher than the display priority 63 of the region images 70 in which the tissue specimens of the subject S having a relatively small deviation 115 of the clinical test value from the normal value are imaged.

In general, the subject S having a relatively large deviation 115 of the clinical test value from the normal value is likely to develop a morphological abnormality as compared with the subject S having a relatively small deviation 115 of the clinical test value from the normal value. Therefore, according to the third embodiment, the probability of being able to collectively observe the region image 70 in which the tissue specimen of the organ in which the morphological abnormality has occurred is imaged with priority is increased. It is possible to contribute to the improvement of the efficiency of the evaluation of the drug efficacy and the toxicity of the candidate substance 27 of the drug and the improvement of the accuracy of the evaluation.

The deviation of the clinical test value from the normal value is not limited to the number of items of the clinical test value indicating the abnormal value illustrated as an example. For example, a difference from the normal value of the clinical test value of a certain specific item may be used as the deviation. In addition to the clinical test value, the display priority 63 may be set with reference to a degree of weight loss of the subject S, a weight of the organ, and the like. For example, the display priority 63 of the region image 70 obtained from the subject S having a relatively large degree of weight loss is set to be high. In addition, the display priority 63 of the region image 70 obtained from the subject S having a relatively heavy weight of the organ is set to be high.

In FIG. 24, an example is shown in which the group of the organ system is not selected in the pull-down menus 86A to 86C, but the present disclosure is not limited to this. The clinical test value button 111 may be selected after the group of the organ system is selected in the pull-down menus 86A to 86C. That is, the first embodiment and the third embodiment may be implemented in combination.

In a case where the clinical test value button 111 is selected after the group of the organ system is selected in the pull-down menus 86A to 86C, the region images 70 are rearranged in the group of the organ system in descending order of the deviation 115 under the control of the display control unit 55.

In addition, the second embodiment and the third embodiment may be implemented in combination. For example, the region images 70 obtained from the subject S of the high-dose group 25H are rearranged in descending order of the deviation 115 from the normal value of the clinical test value.

### [Fourth Embodiment]

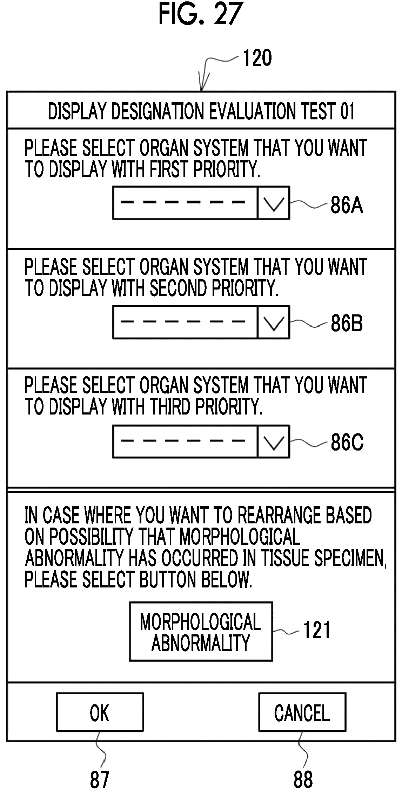

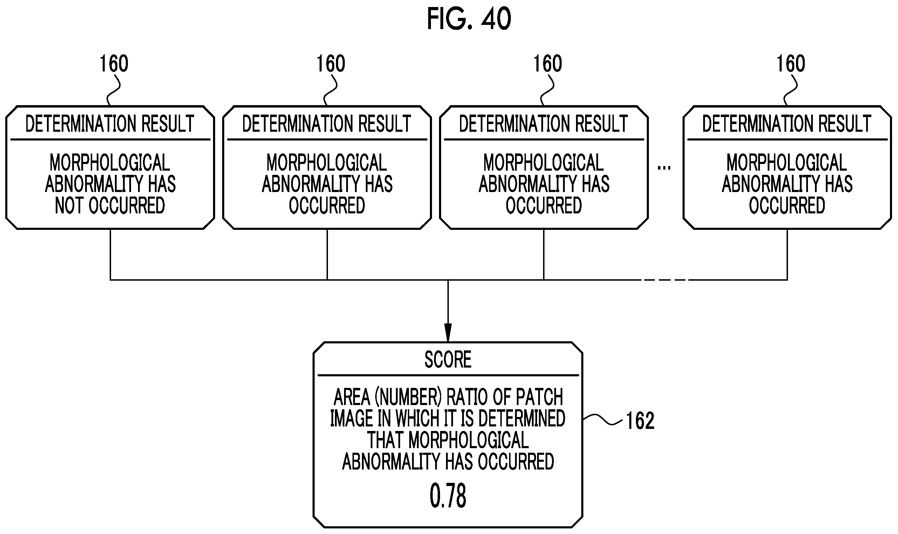

As shown in FIG. 27 as an example, a display designation screen 120 of a fourth embodiment is provided with a morphological abnormality button 121 in addition to the pull-down menus 86A to 86C and the like. In a case where the morphological abnormality button 121 is selected and the OK button 87 is selected by the user U, the instruction reception unit 53 receives the display designation instruction for setting a score 162 (see FIG. 40) representing a possibility that a morphological abnormality has occurred in the tissue specimen imaged in the region image 70 as the criterion for setting the display priority 63 of the region image 70.

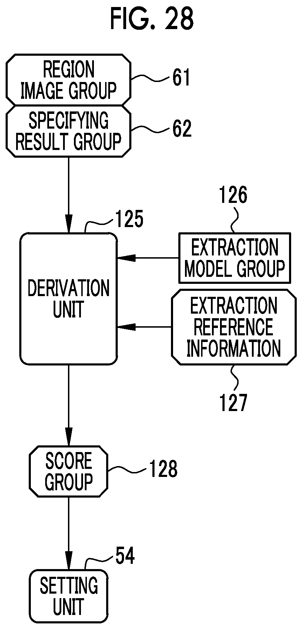

As shown in FIG. 28 as an example, a CPU of an evaluation support apparatus of the fourth embodiment functions as a derivation unit 125 in addition to each of processing units 50 to 55 (units other than the setting unit 54 are not shown) of the first embodiment. The region image group 61 and the specifying result group 62 are input to the derivation unit 125 from the specifying unit 52. In addition, an extraction model group 126 and extraction reference information 127 are input to the derivation unit 125. The extraction model group 126 and the extraction reference information 127 are stored in the storage 30 and are read out from the storage 30 by the RW control unit 50 and input to the derivation unit 125.

The derivation unit 125 extracts a region (hereinafter, referred to as a morphological abnormality occurrence estimation region) in which the morphological abnormality is estimated to have occurred for each of the plurality of region images 70 constituting the region image group 61 by using the extraction model group 126 and the extraction reference information 127. The derivation unit 125 derives the score 162, which is a numerical value based on an area of the morphological abnormality occurrence estimation region, for each of the plurality of region images 70. The derivation unit 125 outputs a score group 128, which is a set of the scores 162 of the plurality of region images 70, to the setting unit 54. In a case where the display designation instruction is received by the instruction reception unit 53, the setting unit 54 sets the display priority 63 of the region image 70 based on the score group 128.

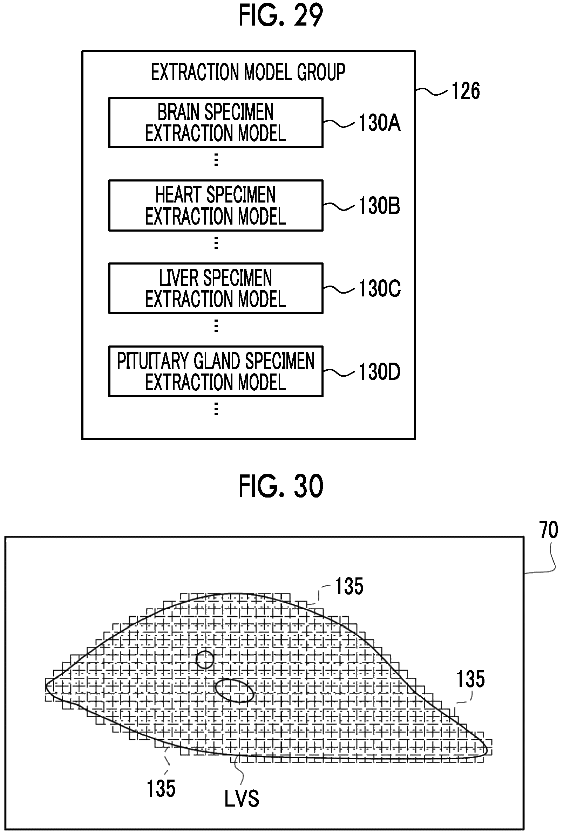

As shown in FIG. 29 as an example, the extraction model group 126 is a set of extraction models 130 prepared for each organ. That is, the extraction model group 126 includes a brain specimen extraction model 130A used in the region image 70 in which the brain specimen BS is imaged, a heart specimen extraction model 130B used in the region image 70 in which the heart specimen HS is imaged, a liver specimen extraction model 130C used in the region image 70 in which the liver specimen LVS is imaged, a pituitary gland specimen extraction model 130D used in the region image 70 in which the pituitary gland specimen PGS is imaged, and the like. The derivation unit 125 uses the extraction model 130 in accordance with the specifying result 75 of the specifying result group 62. The extraction model 130 is an example of a "machine learning model" according to the technology of the present disclosure.

As shown in FIG. 30 as an example, the derivation unit 125 recognizes the tissue specimen (in FIG. 30, the liver specimen LVS is illustrated) imaged in the region image 70 by using a known image recognition technique, and subdivides the recognized tissue specimen into a plurality of patch images 135. The patch image 135 has a preset size that can be handled by the extraction model 130 (in this case, the liver specimen extraction model 130C). The derivation unit 125 assigns a patch image ID to the patch image 135. In addition, the derivation unit 125 associates the patch image ID with information indicating which position of the region image 70 is cut out by the patch image 135, that is, the position information of the patch image 135. In FIG. 30, the patch image 135 does not have a region that overlaps other patch images 135, but the patch image 135 may partially overlap other patch images 135.

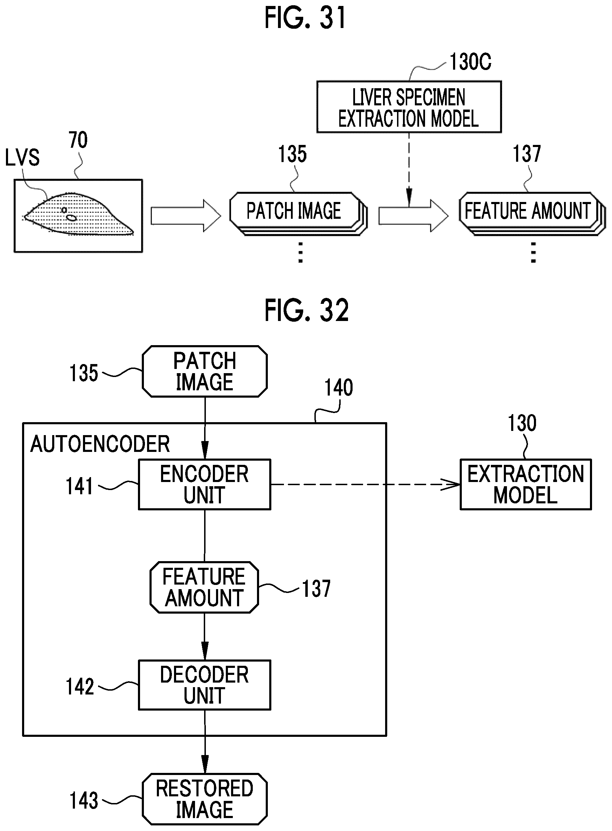

As shown in FIG. 31 as an example, the derivation unit 125 extracts a feature amount 137 for each of the plurality of patch images 135 obtained by subdividing the region image 70 by using the extraction model 130 (in FIG. 31, the liver specimen extraction model 130C). Therefore, the number of feature amounts 137 is the same as the number of patch images 135.

As shown in FIG. 32 as an example, an encoder unit 141 of an autoencoder 140 is used as the extraction model 130. The autoencoder 140 includes a decoder unit 142 in addition to the encoder unit 141. The patch image 135 is input to the encoder unit 141. The encoder unit 141 converts the patch image 135 into the feature amount 137. The encoder unit 141 delivers the feature amount 137 to the decoder unit 142. The decoder unit 142 generates a restored image 143 of the patch image 135 from the feature amount 137.

As is well known, the encoder unit 141 includes a convolutional layer that performs convolution processing using a filter, a pooling layer that performs pooling processing such as maximum value pooling processing, and the like. The same applies to the decoder unit 142. The encoder unit 141 repeatedly performs the convolution processing using the convolutional layer and the pooling processing using the pooling layer on the input patch image 135 a plurality of times to extract the feature amount 137. The extracted feature amount 137 represents a feature of a shape and a texture of the tissue specimen imaged in the patch image 135.

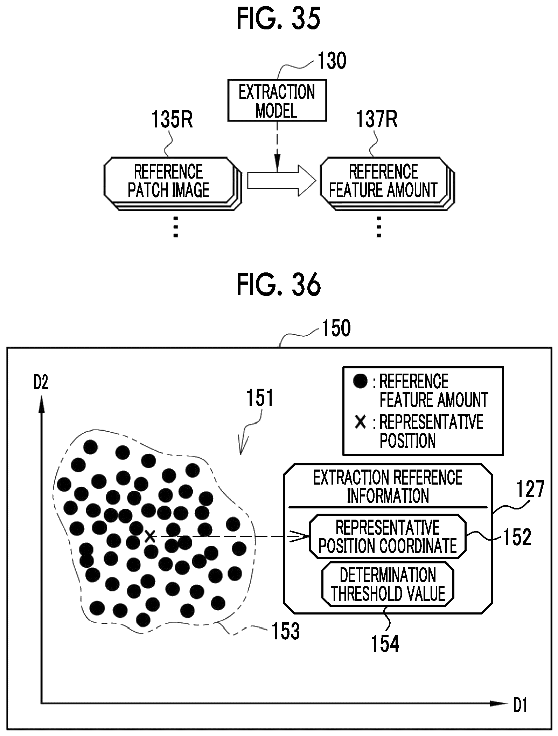

The feature amount 137 is a set of a plurality of numerical values. That is, the feature amount 137 is multi-dimensional data. The number of dimensions of the feature amount 137 is, for example, 512, 1024, or 2048. The feature amount 137 and a reference feature amount 137R (see FIG. 35) described below have the same number of dimensions and can be compared in the same feature amount space 151 (see FIG. 36 and the like).

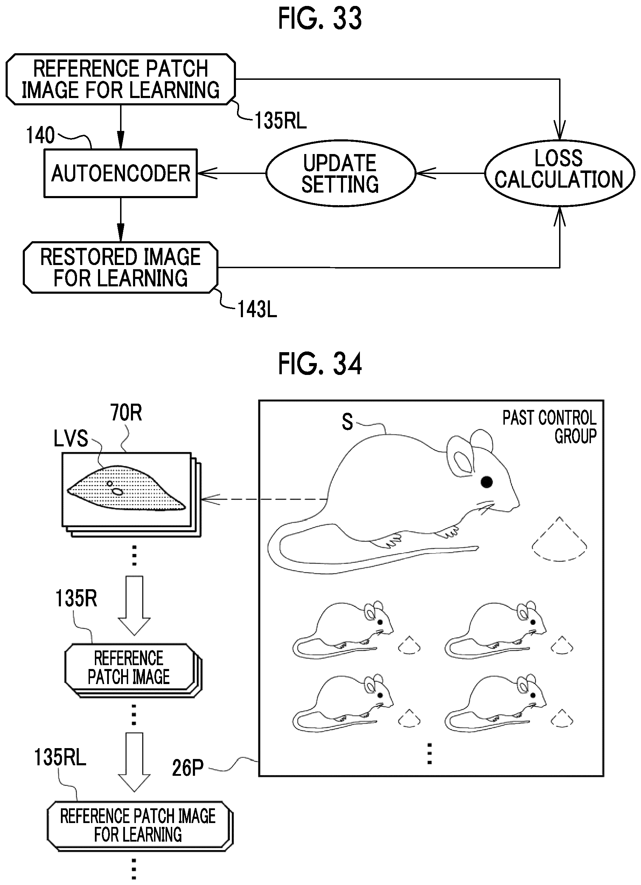

As shown in FIG. 33 as an example, the autoencoder 140 is trained by inputting a reference patch image 135RL for learning in a learning phase before the encoder unit 141 is used as the extraction model 130. The autoencoder 140 outputs a restored image 143L for learning in response to the input of the reference patch image 135RL for learning. The loss calculation of the autoencoder 140 using a loss function is performed based on the reference patch image 135RL for learning and the restored image 143L for learning. Then, the update settings of various coefficients (for example, coefficients of convolutional layer filters) of the autoencoder 140 are performed according to the results of the loss calculation, and the autoencoder 140 is updated according to the update setting.

In the learning phase of the autoencoder 140, the series of processes of the input of the reference patch image 135RL for learning to the autoencoder 140, the output of the restored image 143L for learning from the autoencoder 140, the loss calculation, the update setting, and the update of the autoencoder 140 is repeatedly performed while the reference patch image 135RL for learning is exchanged. The repetition of the series of processes is ended in a case where the restoration accuracy from the reference patch image 135RL for learning to the restored image 143L for learning reaches a predetermined setting level. The encoder unit 141 of the autoencoder 140 in which the restoration accuracy reaches the setting level in this way is stored in the storage 30 of the evaluation support apparatus 10 as the extraction model 130. In addition, in a case where the series of processes is repeated a set number of times, the learning may be ended, regardless of the restoration accuracy from the reference patch image 135RL for learning to the restored image 143L for learning.

The learning of the autoencoder 140 may be performed by the evaluation support apparatus 10 or may be performed by a device different from the evaluation support apparatus 10. In the latter case, the extraction model 130 is transmitted from another device to the evaluation support apparatus 10, and the RW control unit 50 stores the extraction model 130 in the storage 30.

As shown in FIG. 34 as an example, the reference patch image 135RL for learning is supplied from a plurality of reference patch images 135R obtained by subdividing a reference region image 70R. The reference region image 70R is an image in which a tissue specimen of the subject S of a past control group 26P is imaged. The past control group 26P is composed of a plurality of subjects S to which the candidate substance 27 was not administered in the past evaluation test. Therefore, the number of subjects S constituting the past control group 26P is significantly larger than the number of subjects S constituting the dose group 25 and the control group 26, and is, for example, about several hundred to several thousand. Since the reference region image 70R is also obtained in a plurality from one subject S as in the region image 70, the number of reference region images 70R obtained from the past control group 26P is obtained by multiplying the number of reference region images 70R obtained from one subject S by the number of subjects S. It should be noted that, not only the region image 70 in which the tissue specimen of the subject S of the past control group 26P is imaged but also a region image 70 in which a tissue specimen determined to be normal by a specialist such as a pathologist is imaged in a past dose group composed of a plurality of subjects S to which the candidate substance is administered in the past evaluation test may be adopted as the reference region image 70R.

FIG. 34 illustrates the reference region image 70R in which the liver specimen LVS is imaged. The encoder unit 141 of the autoencoder 140 trained by using the reference patch image 135RL for learning based on the reference region image 70R in which the liver specimen LVS is imaged is used as the liver specimen extraction model 130C. Similarly, for example, the encoder unit 141 of the autoencoder 140 trained by using the reference patch image 135RL for learning based on the reference region image 70R in which the brain specimen BS is imaged is used as the brain specimen extraction model 130A.

Next, a configuration of the extraction reference information 127 will be described. First, as shown in FIG. 35 as an example, a plurality of reference feature amounts 137R are extracted from each of a plurality of reference patch images 135R based on all of the plurality of reference region images 70R by using the extraction model 130.

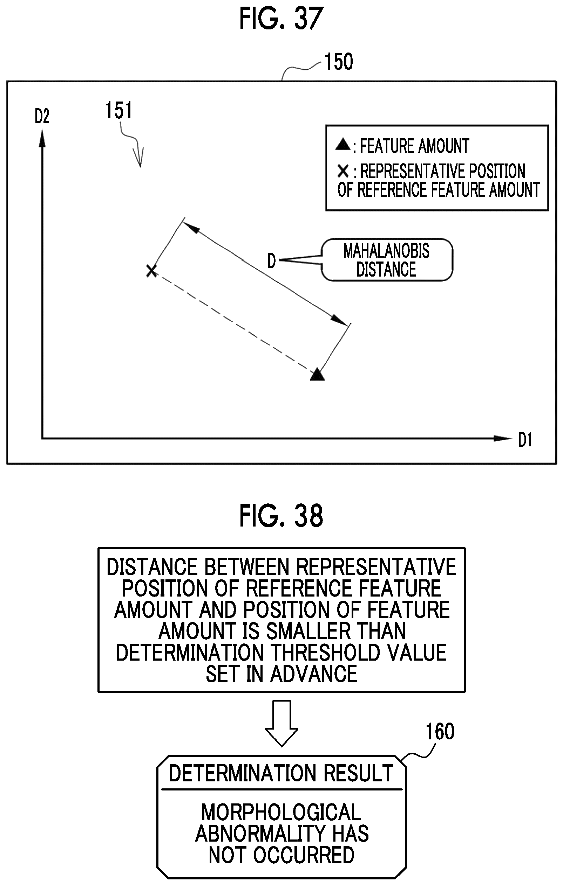

A graph 150 shown in FIG. 36 as an example is a graph in which the plurality of reference feature amounts 137R extracted in FIG. 35 are plotted in the feature amount space 151. The extraction reference information 127 includes coordinates 152 (hereinafter, referred to as representative position coordinates) of a representative position of the reference feature amount 137R indicated by an X mark in the feature amount space 151. The representative position is, for example, a center point or an average point of a distribution 153 of the reference feature amount 137R. In addition, the extraction reference information 127 also includes a determination threshold value 154. In addition, in FIG. 36, for convenience of description, the dimensions of the feature amount space 151 are set to two dimensions having a D1-axis and a D2-axis, but the actual dimensions of the feature amount space 151 are, for example, 512 dimensions as described above. Similarly, in FIG. 37, which will be described below, for convenience of description, the dimension of the feature amount space 151 is represented in two dimensions.

As in the learning of the autoencoder 140, the representative position coordinates 152 of the extraction reference information 127 may be derived by the evaluation support apparatus 10 or may be derived by a device different from the evaluation support apparatus 10. In the latter case, the representative position coordinates 152 are transmitted from another device to the evaluation support apparatus 10, and the RW control unit 50 stores the representative position coordinates 152 in the storage 30.

As shown in FIG. 37 as an example, the derivation unit 125 calculates a distance D between a representative position of the reference feature amount 137R represented by the representative position coordinates 152 of the extraction reference information 127 and a position of the feature amount 137 in the feature amount space 151. The derivation unit 125 calculates the distance D between the plurality of feature amounts 137 extracted for each of the plurality of patch images 135 obtained by subdividing one region image 70. The distance D is a Mahalanobis distance. The distance D indicates a degree of deviation of the feature amount 137 from the reference feature amount 137R, further indicating a degree of deviation of the tissue specimen imaged in the patch image 135 from the tissue specimen considered to be normal. That is, the larger the distance D is, the more the tissue specimen imaged in the patch image 135 deviates from the tissue specimen considered to be normal. Therefore, the larger the distance D is, the higher the possibility that the morphological abnormality has occurred in the tissue specimen imaged in the patch image 135.

As the distance D, any of an average value, a median value, or a maximum value of a Euclidean distance between a position of a k-nearest neighbor sample of the distribution 153 of the reference feature amount 137R and the position of the feature amount 137 may be calculated. Alternatively, instead of the distance D, a value obtained by subtracting a cosine similarity between a vector representing the representative position of the reference feature amount 137R and a vector representing the position of the feature amount 137 from 1.0 may be calculated. The cosine similarity takes a value between -1.0 and 1.0, and it can be said that the larger the value is, the more similar the directions of the vectors are.

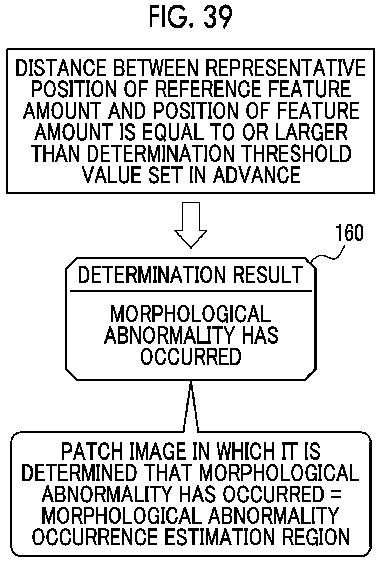

As shown in FIGS. 38 and 39 as an example, the derivation unit 125 compares magnitudes of the calculated distance D and the determination threshold value 154. As shown in FIG. 38, in a case where the distance D is smaller than the determination threshold value 154, the derivation unit 125 determines that the morphological abnormality has not occurred in the tissue specimen imaged in the patch image 135. The derivation unit 125 outputs a determination result 160 indicating that the morphological abnormality has not occurred in the tissue specimen imaged in the patch image 135.

On the other hand, as shown in FIG. 39, in a case where the distance D is equal to or larger than the determination threshold value 154, the derivation unit 125 determines that the morphological abnormality has occurred in the tissue specimen imaged in the patch image 135. The derivation unit 125 outputs the determination result 160 indicating that the morphological abnormality has occurred in the tissue specimen imaged in the patch image 135. The region of the patch image 135 in which it is determined that the morphological abnormality has occurred in the tissue specimen in this way corresponds to the morphological abnormality occurrence estimation region, that is, the "region in which the morphological abnormality is estimated to have occurred" according to the technology of the present disclosure. The determination threshold value 154 may be common to the dose group 25 and the control group 26 or may be different between these groups. In addition, instead of the distance D, it may be determined whether or not the morphological abnormality has occurred in the tissue specimen imaged in the patch image 135 by comparing the cosine similarity and the determination threshold value 154.

As shown in FIGS. 34 and 35, the reference feature amount 137R is a feature amount extracted from the reference patch image 135R obtained by subdividing the reference region image 70R in which the tissue specimen of the subject S of the past control group 26P is imaged. The subject S of the past control group 26P is the subject S to which the candidate substance 27 is not administered. Therefore, at least the morphological abnormality caused by the toxicity of the candidate substance 27 has not occurred in the tissue specimen imaged in the reference region image 70R. Therefore, the representative position of the reference feature amount 137R is regarded as the representative position of the feature amount of the region image 70 in which the normal tissue specimen is imaged. Therefore, as described above, the distance D between the representative position of the reference feature amount 137R and the position of the feature amount 137 is an indicator indicating how much the tissue specimen imaged in the patch image 135 deviates from the normal tissue specimen. Therefore, as shown in FIG. 38, the derivation unit 125 determines that the morphological abnormality has not occurred for the patch image 135 in which the distance D is smaller than the determination threshold value 154, on the assumption that the imaged tissue specimen does not deviate from the normal tissue specimen. On the other hand, as shown in FIG. 39, the derivation unit 125 determines that the morphological abnormality has occurred for the patch image 135 in which the distance D is equal to or larger than the determination threshold value 154, on the assumption that the imaged tissue specimen deviates from the normal tissue specimen.

As shown in FIG. 40 as an example, the derivation unit 125 counts the number of determination results 160 indicating that the morphological abnormality has occurred in the tissue specimen imaged in the patch image 135, that is, the number of morphological abnormality occurrence estimation regions for each of the plurality of region images 70. Then, by dividing the counted number by the total number of the patch images 135, the area (number) ratio of the patch image 135 in which it is determined that the morphological abnormality has occurred, that is, the morphological abnormality occurrence estimation region is calculated as the score 162. The area ratio is an example of a "numerical value based on the area of the region in which the morphological abnormality is estimated to have occurred" according to the technology of the present disclosure. The area (number) of the morphological abnormality occurrence estimation region itself may be output as the score 162 instead of the area ratio.

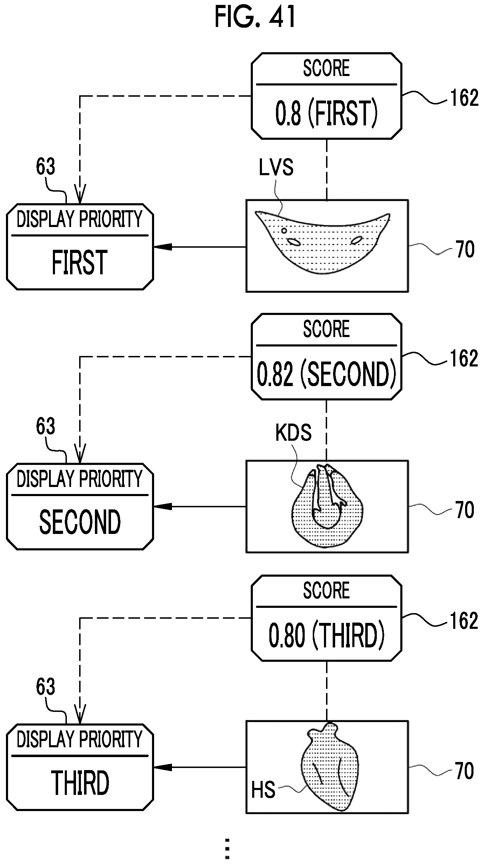

As shown in FIG. 41 as an example, the setting unit 54 sets the display priority 63 of the region image 70 having the first (maximum) score 162 to "first". In addition, the display priority 63 of the region image 70 having the second score 162 is set to "second". Further, the display priority 63 of the region image 70 having the third score 162 is set to "third". Similarly, the setting unit 54 sets the display priority 63 corresponding to the rank of the score 162. That is, the setting unit 54 sets the display priority 63 of the region images 70 in which the tissue specimens having a relatively high score 162 and a relatively high possibility that the morphological abnormality has occurred are imaged to be higher than the display priority 63 of the region images 70 in which the tissue specimens having a relatively low score 162 and a relatively low possibility that the morphological abnormality has occurred are imaged.

As described above, in the fourth embodiment, the derivation unit 125 derives the score 162 representing the possibility that the morphological abnormality has occurred in the tissue specimens imaged in the region images 70. The instruction reception unit 53 receives the designation of the score 162 as the designation of the criterion. The setting unit 54 sets the display priority 63 of the region images 70 in which the tissue specimens having a relatively high score 162 and a relatively high possibility that the morphological abnormality has occurred are imaged to be higher than the display priority 63 of the region images 70 in which the tissue specimens having a relatively low score 162 and a relatively low possibility that the morphological abnormality has occurred are imaged. Therefore, according to the fourth embodiment, the region image 70 in which the tissue specimen of the organ in which the morphological abnormality is estimated to have occurred is imaged can be observed with priority. It is possible to further contribute to the improvement of the efficiency of the evaluation of the drug efficacy and the toxicity of the candidate substance 27 of the drug and the improvement of the accuracy of the evaluation.

The derivation unit 125 extracts the morphological abnormality occurrence estimation region by using the extraction model 130 that is the machine learning model. In recent years, the machine learning model has made remarkable progress, and it is possible to easily prepare a relatively high-accuracy machine learning model. Therefore, it is possible to easily and accurately extract the morphological abnormality occurrence estimation region.

The score 162 is a numerical value based on the area of the morphological abnormality occurrence estimation region, and is an area ratio of the morphological abnormality occurrence estimation region in the example. Therefore, the validity of the score 162 and the display priority 63 can be increased as compared with a case where a rough numerical value indicating the probability of whether or not the morphological abnormality has occurred in the tissue specimen is set as the score 162.

In FIG. 27, an example is shown in which the group of the organ system is not selected in the pull-down menus 86A to 86C, but the present disclosure is not limited to this. The morphological abnormality button 121 may be selected after the group of the organ system is selected in the pull-down menus 86A to 86C. That is, the first embodiment and the fourth embodiment may be implemented in combination.

In a case where the morphological abnormality button 121 is selected after the group of the organ system is selected in the pull-down menus 86A to 86C, the region images 70 are rearranged in the group of the organ system in descending order of the score 162 under the control of the display control unit 55.

In addition, the second embodiment and the fourth embodiment may be implemented in combination. For example, the region images 70 obtained from the subject S of the high-dose group 25H are rearranged in descending order of the score 162.

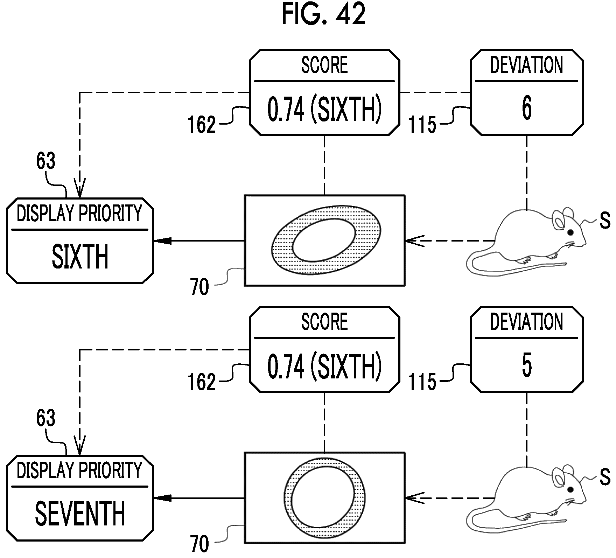

Further, as shown in FIG. 42 as an example, the third embodiment and the fourth embodiment may be implemented in combination. That is, for the two region images 70 having the same score 162, the display priority 63 of the region image 70 obtained from the subject S having the relatively large deviation 115 is set to be higher than the display priority 63 of the region image 70 obtained from the subject S having the relatively small deviation 115. In this way, the display priority 63 of the plurality of region images 70 having the same score 162 can be set by assigning an order, without being the same.

In addition, unlike the aspect shown in FIG. 42, for the two region images 70 having the same deviation 115, the display priority 63 of the region image 70 having the relatively high score 162 may be set to be higher than the display priority 63 of the region image 70 having the relatively low score 162.

In addition to the reference patch image 135R in which the tissue specimen considered to be normal is imaged, the patch image 135 in which the tissue specimen in which the morphological abnormality has occurred is imaged may be used as the reference patch image 135RL for learning. The patch image 135 in which the tissue specimen in which the morphological abnormality has occurred is imaged is acquired from, for example, a past dose group composed of a plurality of subjects S to which the candidate substance 27 is administered in the past evaluation test. As a result, the autoencoder 140 and the extraction model 130 can be trained for the tissue specimen having a more diverse feature of the shape and the texture. As a result, the extraction model 130 can extract the feature amount 137 that better represents the feature of the shape and the texture of the tissue specimen.

The patch image 135 in which the tissue specimen in which the morphological abnormality has occurred is imaged is not limited to the one acquired from the subject S constituting the past dose group illustrated as an example. In the subject S constituting the past control group 26P, the morphological abnormality may also occur. Therefore, in a case where the patch image 135 in which the tissue specimen in which the morphological abnormality has occurred is imaged, it does not matter whether the subject S is the past control group 26P or the past dose group. Further, the patch image 135 in which the tissue specimen in which the morphological abnormality has occurred is imaged may be an image acquired from the subject S that is designed to develop the morphological abnormality by applying various stresses. In addition, the patch image 135 in which the tissue specimen in which the morphological abnormality has occurred is imaged may be an image artificially created by processing the patch image 135 in which the normal tissue specimen is imaged.

An encoder unit of a convolutional neural network that outputs a class discrimination result in response to the input of the patch image 135 may be used as the extraction model 130 instead of the encoder unit 141 of the autoencoder 140. The class discrimination result is, for example, a result of discriminating one type of morphological abnormality that has occurred in the tissue specimen imaged in the patch image 135 from among a plurality of types such as hyperplasia, infiltration, congestion, and inflammation.

In addition, the machine learning model to be used as the extraction model 130 is not limited to the autoencoder 140 and the convolutional neural network illustrated as an example. A generator of a generative adversarial network (GAN) may be used as the extraction model 130. A machine learning model that does not have a convolutional layer, such as a vision transformer (ViT), may be used as the extraction model 130.

Contrastive learning of bringing the distance between the feature amounts derived from the same image closer to each other in the feature amount space and moving the distance between the feature amounts derived from different images farther from each other in the feature amount space may be performed. As the contrastive learning, for example, a learning method such as a simple framework for contrastive learning of visual representations (SimCLR) is known. In addition, a learning method such as bootstrap your own latent (BYOL) that does not use the above-described pair of different images (also referred to as a negative sample) may be used. In addition, a constraint such as a distribution on a unit sphere or a distribution following a standard normal distribution may be applied to the distribution of the feature amount to be extracted.

The feature amount 137 is not limited to the feature amount extracted by the extraction model 130. The feature amount 137 may be, for example, the average value, maximum value, minimum value, mode value, or variance of the pixel values of the patch image 135.

Only the region image 70 in which the display priority 63 is set to "high" or "first" may be selectively displayed on the image list display screen 80. In addition, the target of the region image 70 to be displayed on the image list display screen 80 may be limited to only one subject S, only the high-dose group 25H, only the medium-period dose group 25MT, or the like.

In each of the above-described embodiments, a case where one slide specimen 18 has a plurality of tissue specimens has been illustrated as an example, but the present disclosure is not limited to this. The technology of the present disclosure can also be applied to a case where one slide specimen 18 has one tissue specimen.

The subject S is not limited to the rat. The subject S may be a mouse, a guinea pig, a sand mouse, a hamster, a ferret, a rabbit, a dog, a cat, a monkey, or the like. In addition, the subject S may be a human. In a case where the subject S is the human, the display priority 63 of the region image 70 may be set based on genetic information. For example, the display priority 63 of the region image 70 obtained from the subject S having a genetic feature that is likely to develop a morphological abnormality in the genetic information is set to be higher than the display priority 63 of the other region image 70.

The evaluation support apparatus 10 may be a personal computer that is installed in a pharmaceutical facility as shown in FIG. 1 or may be a server computer that is installed in a data center independent of the pharmaceutical facility.

In a case where the evaluation support apparatus 10 is configured by the server computer, the specimen image 15 is transmitted from the personal computer installed in each pharmaceutical facility to the server computer via a network such as the Internet. The server computer distributes various screens, such as the image list display screen 80, to the personal computer, for example, in a format of screen data for web distribution created by a markup language such as extensible markup language (XML). The personal computer reproduces a screen displayed on a web browser based on the screen data and displays the reproduced screen on the display. Note that, instead of XML, another data description language, such as JavaScript (registered trademark) Object Notation (JSON), may be used.

The evaluation support apparatus 10 according to the technology of the present disclosure can be widely used in all stages of pharmaceutical development from the setting of a drug discovery target in the earliest stage to the clinical trial in the final stage.

The hardware configuration of the computer constituting the evaluation support apparatus 10 according to the technology of the present disclosure can be modified in various ways. For example, the evaluation support apparatus 10 may be configured by a plurality of computers that are separated as hardware for the purpose of improving processing capacity and reliability. For example, the functions of the identification unit 51 and the specifying unit 52 and the function of the setting unit 54 are distributed to two computers. In this case, the evaluation support apparatus 10 is configured by the two computers.

As described above, the hardware configuration of the computer of the evaluation support apparatus 10 can be changed as appropriate depending on required performance such as processing capacity, safety, and reliability. Further, it goes without saying that, in addition to the hardware, an application program such as the operation program 40 can be duplicated or distributed and stored in a plurality of storages for the purpose of ensuring the safety and the reliability.

In each of the above-described embodiments, for example, the following various processors described below can be used as a hardware structure of processing units that execute various types of processing, such as the RW control unit 50, the identification unit 51, the specifying unit 52, the instruction reception unit 53, the setting unit 54, the display control unit 55, and the derivation unit 125. The various processors include, for example, in addition to the CPU 32 that is a general-purpose processor executing software (operation program 40) to function as various processing units as described above, a programmable logic device (PLD) that is a processor of which a circuit configuration can be changed after manufacture, such as a field programmable gate array (FPGA), and a dedicated electric circuit that is a processor having a dedicated circuit configuration designed to execute a specific process, such as an application specific integrated circuit (ASIC).

One processing unit may be configured by one of the various types of processors or may be configured by a combination of two or more processors of the same type or different types (for example, a combination of a plurality of FPGAs and/or a combination of a CPU and an FPGA). In addition, a plurality of processing units may be configured by one processor.

As an example of configuring the plurality of processing units with one processor, first, there is a form in which one processor is configured by a combination of one or more CPUs and software and the processor functions as the plurality of processing units, as represented by computers such as a client and a server. A second example of the configuration is a form in which a processor that implements the functions of the entire system including the plurality of processing units using one integrated circuit (IC) chip is used, as represented by a system on chip (SoC). As described above, the various processing units are configured by using one or more of the above various processors as the hardware structure.

In addition, more specifically, an electric circuit (circuitry) in which circuit elements, such as semiconductor elements, are combined can be used as the hardware structure of these various processors.

It is possible to understand the technology described in the following supplementary notes from the above description.

### [Supplementary Note 1]

An image display device comprising:
a processor,
in which the processor is configured to:
   acquire a plurality of specimen images in which tissue specimens of a subject provided for an evaluation test of a candidate substance of a drug are imaged;
   receive designation of a criterion for setting a display priority of the plurality of specimen images from a user;
   set the display priority in accordance with the designated criterion; and
   perform control of displaying the plurality of specimen images on a display unit in accordance with the set display priority.

### [Supplementary Note 2]

The image display device according to Supplementary Note 1,
in which organs serving as sources of the tissue specimens are of a plurality of types,
the plurality of types of organs are classified into a plurality of groups in advance, and
the processor is configured to:
   receive designation of the group as the designation of the criterion;
   specify types of the organs of the tissue specimens imaged in the specimen images; and
   set a display priority of the specimen images in which the tissue specimens of the organs belonging to the designated group are imaged to be higher than a display priority of the specimen images in which the tissue specimens of the organs belonging to a group other than the designated group are imaged.

### [Supplementary Note 3]

The image display device according to Supplementary Note 2,
in which the processor is configured to perform control of displaying the specimen images collectively for each group.

### [Supplementary Note 4]

The image display device according to Supplementary Note 2 or 3,
in which a plurality of the tissue specimens are imaged in one specimen image, and
the processor is configured to:
   identify the plurality of tissue specimens imaged in the one specimen image; and
   specify types of the organs of the identified tissue specimens.

### [Supplementary Note 5]

The image display device according to Supplementary Note 4,
in which the processor is configured to:
generate region images of the identified tissue specimens from the specimen image; and
rearrange the region images in accordance with the set display priority.

### [Supplementary Note 6]

The image display device according to any one of Supplementary Notes 2 to 5,
in which the group is a group based on an organ system.

### [Supplementary Note 7]

The image display device according to any one of Supplementary Notes 2 to 5,
in which the group is a group based on knowledge of the user.

### [Supplementary Note 8]

The image display device according to any one of Supplementary Notes 1 to 7,
in which the processor is configured to:
receive designation of any one of a dose, a dosing period, or a dosing frequency of the candidate substance to the subject as the designation of the criterion; and
set a display priority of the specimen images in which the tissue specimens of the subject having a relatively large dose, a relatively long dosing period, or a relatively high dosing frequency are imaged to be higher than a display priority of the specimen images in which the tissue specimens of the subject having a relatively small dose, a relatively short dosing period, or a relatively low dosing frequency are imaged.

### [Supplementary Note 9]

The image display device according to any one of Supplementary Notes 1 to 8,
in which the processor is configured to:
receive designation of a clinical test value of the subject as the designation of the criterion; and
set a display priority of the specimen images in which the tissue specimens of the subject having a relatively large deviation of the clinical test value from a normal value are imaged to be higher than a display priority of the specimen images in which the tissue specimens of the subject having a relatively small deviation of the clinical test value from the normal value are imaged.

### [Supplementary Note 10]

The image display device according to any one of Supplementary Notes 1 to 9,
in which the processor is configured to:
derive a score representing a possibility that a morphological abnormality has occurred in the tissue specimens imaged in the specimen images;
receive designation of the score as the designation of the criterion; and
set a display priority of the specimen images in which the tissue specimens having a relatively high score and a relatively high possibility that the morphological abnormality has occurred are imaged to be higher than a display priority of the specimen images in which the tissue specimens having a relatively low score and a relatively low possibility that the morphological abnormality has occurred are imaged.

### [Supplementary Note 11]

The image display device according to Supplementary Note 10,
in which the processor is configured to extract a region in which the morphological abnormality is estimated to have occurred by using a machine learning model.

### [Supplementary Note 12]

The image display device according to Supplementary Note 10 or 11,
in which the score is a numerical value based on an area of the region in which the morphological abnormality is estimated to have occurred.

The above various embodiments and/or various modification examples can be combined as appropriate in the technology of the present disclosure. In addition, it goes without saying that the present disclosure is not limited to each of the embodiments described above, and various configurations can be adopted without departing from the gist. Furthermore, the technology of the present disclosure extends to a storage medium that non-transitorily stores the program, and a computer program product including the program, in addition to the program.

The above description content and illustrated content are detailed descriptions of portions related to the technology of the present disclosure and are merely examples of the technology of the present disclosure. For example, the above description of the configurations, functions, operations, and effects is the description of examples of the configurations, functions, operations, and effects of portions according to the technology of the present disclosure. Therefore, it is needless to say that unnecessary portions may be deleted or new elements may be added or replaced in the above description content and illustrated content without departing from the gist of the technology of the present disclosure. In addition, in the above description content and illustrated content, the description of, for example, common technical knowledge that does not need to be particularly described to enable the implementation of the technology of the present disclosure is omitted in order to avoid confusion and facilitate the understanding of portions related to the technology of the present disclosure.

In the present specification, "A and/or B" is synonymous with "at least one of A or B". That is, "A and/or B" may mean only A, only B, or a combination of A and B. Further, in the present specification, the same concept as "A and/or B" is also applied to a case where three or more matters are linked and expressed by "and/or".

All documents, patent applications, and technical standards described in the present specification are incorporated in the present specification by reference to the same extent as in a case where each of the documents, patent applications, and technical standards are specifically and individually indicated to be incorporated by reference.

## Claims

1. An image display device comprising:
a processor,
wherein the processor is configured to:
acquire a plurality of specimen images in which tissue specimens of a subject provided for an evaluation test of a candidate substance of a drug are imaged;
receive designation of a criterion for setting a display priority of the plurality of specimen images from a user;
set the display priority in accordance with the designated criterion; and
perform control of displaying the plurality of specimen images on a display unit in accordance with the set display priority.

2. The image display device according to claim 1,
wherein organs serving as sources of the tissue specimens are of a plurality of types,
the plurality of types of organs are classified into a plurality of groups in advance, and
the processor is configured to:
receive designation of the group as the designation of the criterion;
specify types of the organs of the tissue specimens imaged in the specimen images; and
set a display priority of the specimen images in which the tissue specimens of the organs belonging to the designated group are imaged to be higher than a display priority of the specimen images in which the tissue specimens of the organs belonging to a group other than the designated group are imaged.

3. The image display device according to claim 2,
wherein the processor is configured to perform control of displaying the specimen images collectively for each group.

4. The image display device according to claim 2,
wherein a plurality of the tissue specimens are imaged in one specimen image, and
the processor is configured to:
identify the plurality of tissue specimens imaged in the one specimen image; and
specify types of the organs of the identified tissue specimens.

5. The image display device according to claim 4,
wherein the processor is configured to:
generate region images of the identified tissue specimens from the specimen image; and
rearrange the region images in accordance with the set display priority.

6. The image display device according to claim 2,
wherein the group is a group based on an organ system.

7. The image display device according to claim 2,
wherein the group is a group based on knowledge of the user.

8. The image display device according to claim 1,
wherein the processor is configured to:
receive designation of any one of a dose, a dosing period, or a dosing frequency of the candidate substance to the subject as the designation of the criterion; and
set a display priority of the specimen images in which the tissue specimens of the subject having a relatively large dose, a relatively long dosing period, or a relatively high dosing frequency are imaged to be higher than a display priority of the specimen images in which the tissue specimens of the subject having a relatively small dose, a relatively short dosing period, or a relatively low dosing frequency are imaged.

9. The image display device according to claim 1,
wherein the processor is configured to:
receive designation of a clinical test value of the subject as the designation of the criterion; and
set a display priority of the specimen images in which the tissue specimens of the subject having a relatively large deviation of the clinical test value from a normal value are imaged to be higher than a display priority of the specimen images in which the tissue specimens of the subject having a relatively small deviation of the clinical test value from the normal value are imaged.

10. The image display device according to claim 1,
wherein the processor is configured to:
derive a score representing a possibility that a morphological abnormality has occurred in the tissue specimens imaged in the specimen images;
receive designation of the score as the designation of the criterion; and
set a display priority of the specimen images in which the tissue specimens having a relatively high score and a relatively high possibility that the morphological abnormality has occurred are imaged to be higher than a display priority of the specimen images in which the tissue specimens having a relatively low score and a relatively low possibility that the morphological abnormality has occurred are imaged.

11. The image display device according to claim 10,
wherein the processor is configured to extract a region in which the morphological abnormality is estimated to have occurred by using a machine learning model.

12. The image display device according to claim 10,
wherein the score is a numerical value based on an area of the region in which the morphological abnormality is estimated to have occurred.

13. An operation method of an image display device, the operation method comprising:
acquiring a plurality of specimen images in which tissue specimens of a subject provided for an evaluation test of a candidate substance of a drug are imaged;
receiving designation of a criterion for setting a display priority of the plurality of specimen images from a user;
setting the display priority in accordance with the designated criterion; and
performing control of displaying the plurality of specimen images on a display unit in accordance with the set display priority.

14. An operation program of an image display device, the operation program causing a computer to execute a process comprising:
acquiring a plurality of specimen images in which tissue specimens of a subject provided for an evaluation test of a candidate substance of a drug are imaged;
receiving designation of a criterion for setting a display priority of the plurality of specimen images from a user;
setting the display priority in accordance with the designated criterion; and
performing control of displaying the plurality of specimen images on a display unit in accordance with the set display priority.
